(19) Europäisches Patentamt
European Patent Office
Office européen des brevets

(11) Publication number: **0 095 029**
**B1**

(12) # EUROPEAN PATENT SPECIFICATION

(45) Date of publication of patent specification: **05.11.86**

(21) Application number: **83103110.9**

(22) Date of filing: **25.11.80**

(60) Publication number of the earlier application in accordance with Art. 76 EPC: **0 029 998**

(51) Int. Cl.[4]: **C 07 D 257/04** // C07D209/48, C07D501/18, C07D501/36, C07D295/12, C07D295/20, C07C159/00

(54) **5-Thio-tetrazol compounds and their preparation.**

(30) Priority: **30.11.79 GB 7941417**
**17.12.79 GB 7943363**

(43) Date of publication of application:
**30.11.83 Bulletin 83/48**

(45) Publication of the grant of the patent:
**05.11.86 Bulletin 86/45**

(84) Designated Contracting States:
**AT BE CH DE FR GB IT LI NL SE**

(56) References cited:
**FR-A-2 249 882**
**FR-A-2 295 751**

(73) Proprietor: **Fujisawa Pharmaceutical Co., Ltd.**
**3, Doshomachi 4-chome Higashi-ku**
**Osaka-shi, Osaka 541 (JP)**

(72) Inventor: **Takaya, Takao**
**No. 1-5-87, Suimeidai**
**Kawanishi (JP)**
Inventor: **Inoue, Yoshikazu**
**No. 233-3, Aza-Minamidai**
**Shimokema Amagasaki (JP)**
Inventor: **Yasuda, Nobuyoshi**
**10-12, Kitashowa-cho**
**Nishinomiya (JP)**
Inventor: **Murata, Masayoshi**
**No. 7-7-17, Higashitokiwadai Toyono-cho**
**Toyono-gun Osaka-fu (JP)**
Inventor: **Ueda, Ikuo**
**No. 4-13-36, Kumano-cho**
**Toyanaka (JP)**
Inventor: **Matsuo, Masaaki**
**No. 5-4-12, Nakasakurazuka**
**Toyonaka (JP)**
Inventor: **Tsuji, Kiyoshi**
**No. 170, Hata-cho**
**Kishiwada (JP)**
Inventor: **Kato, Masayuki**
**No. 3-11-30, Mino**
**Mino (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 095 029 B1

(74) Representative: **Türk, Dietmar, Dr. rer. nat. et al**
**Türk, Gille + Hrabal Patentanwälte Bruckner Strasse 20**
**D-4000 Düsseldorf 13 (DE)**

**Description**

The present invention relates to new starting compounds for preparing cephem compounds and processes for their preparation. The cephem compounds prepared therefrom and pharmaceutically acceptable salts thereof have antimicrobial activities as described and claimed in EP—A—80 107 350.3 (EP—B—29998) and are useful in treatment of infectious diseases in human being and animals.

Accordingly, one object of the present invention is to provide new starting compounds for preparing cephem compounds, the starting compounds having the formula:

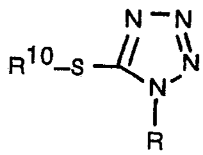

(III′)

wherein R is a group of the formula: $—A_1—R^{3a}$

[in which

$A_1$ is hydroxy($C_1—C_6$)alkylene, amino($C_1—C_6$)alkylene, protected amino($C_1—C_6$)alkylene, alkyenylene with up to 6C-atoms;

or hydroxyimino($C_1—C_6$)alkylene wherein the hydrogen atom of the hydroxyimino group may be replaced with a ($C_1—C_6$)aliphatic hydrocarbon group; and

$R^{3a}$ is carboxy or an esterified carboxy group] or

a group of the formula:

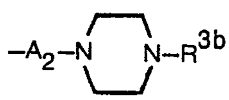

[in which

$A_2$ is ($C_1—C_6$)alkylene which may have an oxo group; and

$R^{3b}$ is carboxy, esterified carboxy, an aliphatic or aromatic group] and

$R^{10}$ is hydrogen or a mercapto-protective group, and a salt thereof.

Another object of the present invention is to provide processes for preparing the same.

The present application is a divisional application from EP—A—80 107 350.3, which discloses and claims novel cephem compounds having the general formula (I):

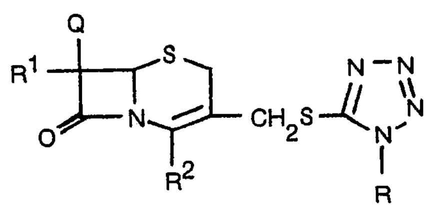

(I)

wherein

$R^1$ is amino or an acylamino group;

$R^2$ is carboxy or an esterified carboxy group;

R is as defined above; and

Q is hydrogen or ($C_1—C_6$)alkoxy.

Starting from the compounds of the present invention, the new cephem compounds (I) can be prepared by the following processes which is illustrated in the following scheme.

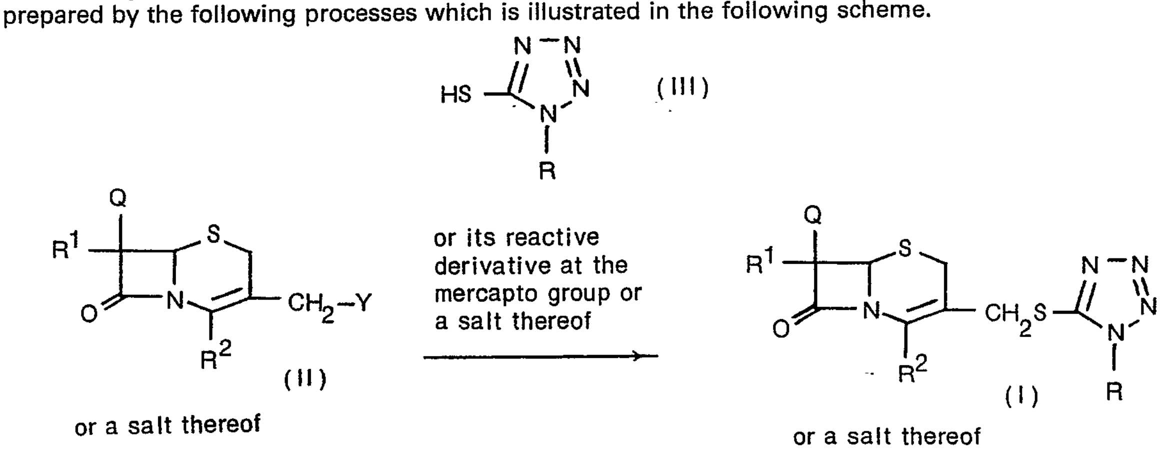


or a salt thereof

or a salt thereof

wherein

$R^1$, $R^2$, R and Q are each as defined above,

Y is a group which can be substituted by a group of the formula:

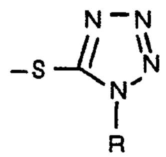

(wherein R is as defined above).

The compounds (III) of the present invention are novel and can be prepared by the processes which are illustrated in the following scheme.

PROCESS A

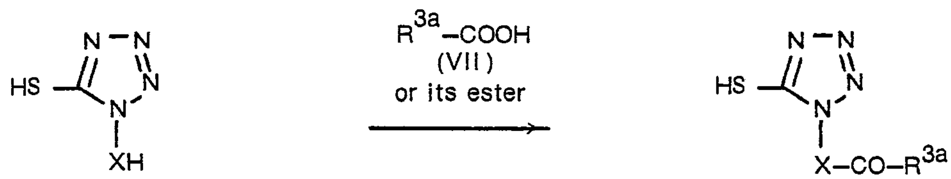


(V)

or its reactive derivative at the mercapto group

(IV)

or its reactive derivative at the mercapto group or a salt thereof

PROCESS B

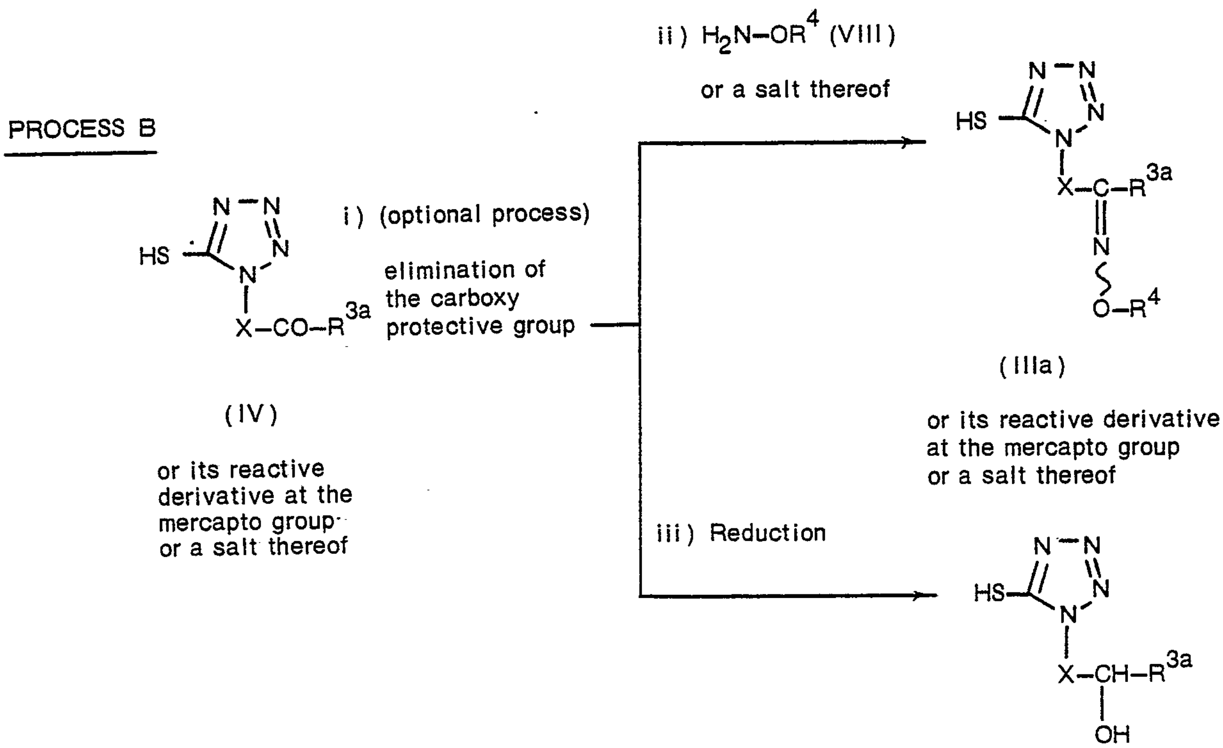


(IV)

or its reactive derivative at the mercapto group or a salt thereof

(IIIa)

or its reactive derivative at the mercapto group or a salt thereof

(IIIb)

or its reactive derivative at the mercapto group or a salt thereof

PROCESS C

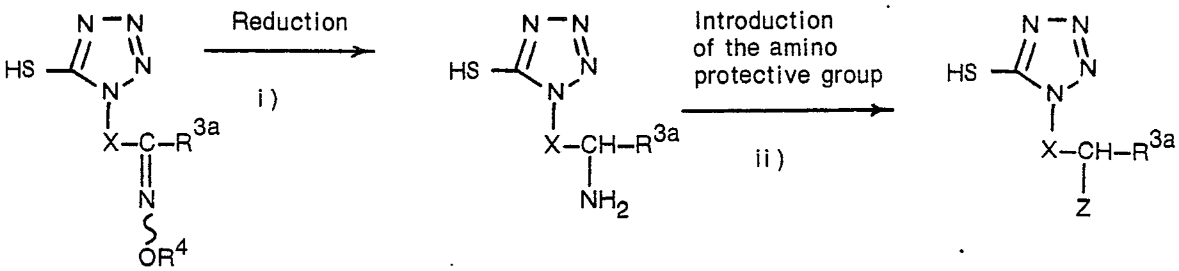


(IIIa)

or its reactive derivative
at the mercapto group
or a salt thereof

(IIId)

or its reactive derivative
at the mercapto group
or a salt thereof

(IIIc)

or its reactive derivative
at the mercapto group
or a salt thereof

PROCESS D

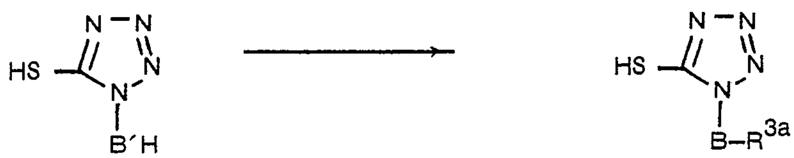

(IIIf)

or its reactive-derivative
at the mercapto group

(IIIe)

or its reactive derivative
at the mercapto group
or a salt thereof

PROCESS E

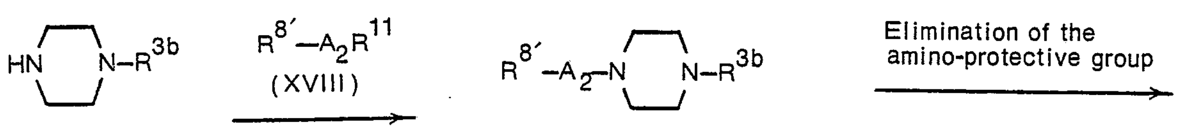


(XIX)

or a salt thereof

(XVII)

or a salt thereof

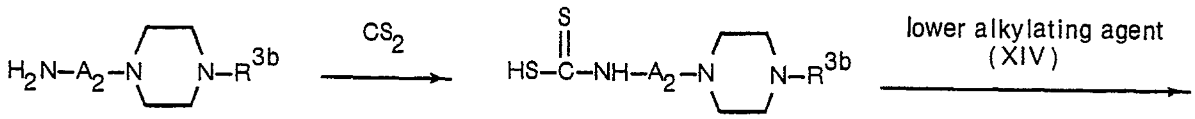


(XVI)

or a salt thereof

(XV)

or a salt thereof

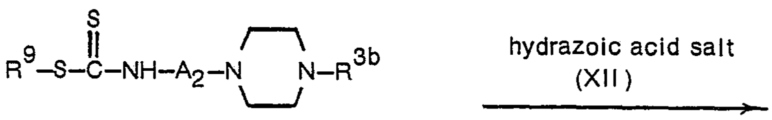


(XIII)

or a salt thereof

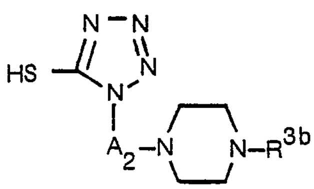

(IIIg)

or a salt thereof

PROCESS F

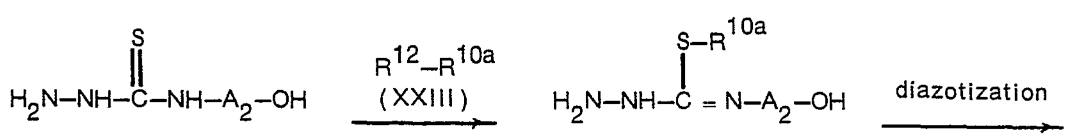


(XXIV)

or a salt thereof

(XXII)

or a salt thereof

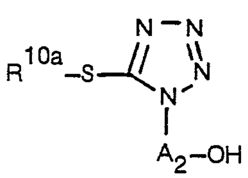

(XXI)

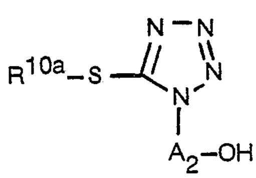

(XXI)

or its reactive derivative at the hydroxy group

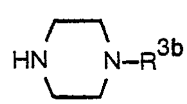

(XIX)

or a salt thereof

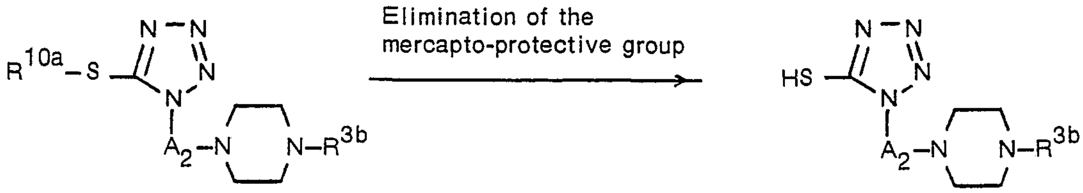


(XX)

or a salt thereof

(IIIg)

or a salt thereof

PROCESS G

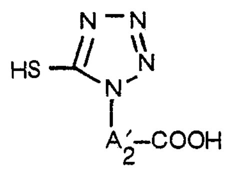

(XXV)

or its reactive derivative
at the carboxy group
or a salt thereof

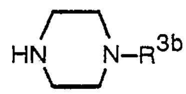

(XIX)

or its reactive derivative
at the amino group
or a salt thereof

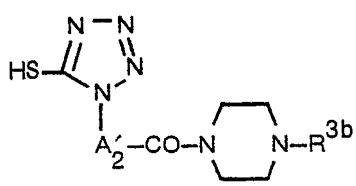

(IIIi)

or a salt thereof

wherein

$R^{3a}$, $R^{3b}$ and $A_2$ are each as defined above;

X is $(C_1—C_6)$alkylene;

$R^4$ is hydrogen or a $(C_1—C_6)$aliphatic hydrocarbon group;

Z is a protected amino group;

B is $(C_2—C_6)$alkenylene;

B' is $(C_2—C_6)$alkenylene;

$R^{8'}$ is amino having a protective group;

$R^9$ is $(C_1—C_6)$alkyl;

$R^{10a}$ is a mercapto-protective group;

$A'_2$ is $(C_1—C_6)$alkylene; and

$R^{11}$ and $R^{12}$ are each an acid residue.

In the above and subsequent descriptions of the present specification, suitable examples and illustrations of the various definitions which the present invention include within the scope thereof are explained in details as follows.

The term "lower" is intended to mean 1 to 6 carbon atoms, unless otherwise indicated.

Suitable "protected amino" may include an acylamino or an amino group substituted by a conventional protecting group such as ar(lower)alkyl which may have at least one suitable substituent(s), (e.g. benzyl, trityl, etc.) or the like.

Suitable "acyl" and "acyl moiety" in the term "acylamino" may include carbamoyl, an aliphatic acyl group, an acyl group containing an aromatic ring (hereinafter referred to as aromatic acyl) and an acyl group containing a heterocyclic ring (hereinafter referred to as heterocyclic acyl).

Suitable example of said acyl may be illustrated as follows:—

Aliphatic acyl such as lower or higher alkanoyl (e.g. formyl, acetyl, succinyl, hexanoyl, heptanoyl, stearoyl, etc.); lower or higher alkoxycarbonyl (e.g. methoxycarbonyl, ethoxycarbonyl, tert-butoxycarbonyl, tert-pentyloxycarbonyl, heptyloxycarbonyl, etc.); lower or higher alkanesulfonyl (e.g. methanesulfonyl, ethanesulfonyl, etc.); or the like.

Aromatic acyl such as aroyl (e.g. benzoyl, toluoyl, naphthoyl, etc.); ar(lower)alkanoyl (e.g. phenylacetyl, phenylpropionyl, etc.); aryloxycarbonyl (e.g. phenoxycarbonyl, naphthyloxycarbonyl, etc.); ar(lower)alkoxycarbonyl (e.g. benzyloxycarbonyl, phenethyloxycarbonyl, etc.); aryloxy(lower)alkanoyl (e.g. phenoxyacetyl, phenoxypropionyl, etc.); arylglyoxyloyl (e.g. phenylglyoxyloyl, naphthylglyoxyloyl, etc.); arenesulfonyl (e.g. benzenesulfonyl, p-toluenesulfonyl, etc.); or the like;

Heterocyclic acyl such as heterocycliccarbonyl (e.g. thenoyl, furoyl, nicotinoyl, etc.); heterocyclic(lower)alkanoyl (e.g. thienylacetyl, thiazolylacetyl, thiadiazolylacetyl, dithiinylacetyl, pyridylacetyl, pyrimidinylacetyl, triazolylacetyl, tetrazolylacetyl, furylacetyl, oxazolylacetyl, thiazolylpropionyl, etc.); heterocyclicglyoxyloyl (e.g. thiazolylglyoxyloyl, thienylglyoxyloyl, etc.); or the like.

The acyl and acyl moiety as stated above may have one or more, same or different, suitable substituent(s) such as lower alkyl as mentioned below; lower alkoxy (e.g. methoxy, ethoxy, propoxy, etc.); lower alkylthio (e.g. methylthio, ethylthio, etc.); lower alkylamino (e.g. methylamino, etc.); halogen (e.g. chlorine, bromine, fluorine or iodine); amino; a protected amino gorup as mentioned above; hydroxy; a protected hydroxy such as tetrahydropyranyloxy or acyloxy wherein the acyl moiety is as stated above; imino; oxo; carboxy, protected carboxy as mentioned below; a group of the formula: $=N—OR^6$ wherein $R^6$ is an aliphatic or aromatic group; or the like.

With regard to compounds (III) and (IIIa), it is also to be noted that where the oxyimino group(s) is contained in the chemical structure thereof, and these compounds exist as geometrical isomers, i.e. syn isomer, anti isomer and a mixture thereof.

The syn isomer means one geometrical isomer having the partial structure represented by the following formula:

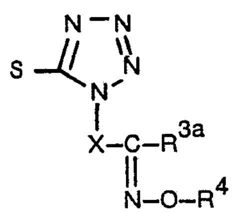

(wherein $R^{3a}$, $R^4$ and X are each as defined above) and the antiisomer means one geometrical isomer having the partial structure represented by the following formula:

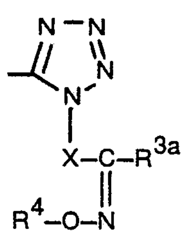

(wherein $R^{3a}$, $R^4$ and X are each as defined above).

The formula:

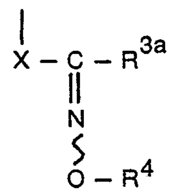

(wherein $R^{3a}$, $R^4$ and X are each as defined above) are intended to represent both of the syn isomer and anti isomer as well as a mixture thereof and both of the respective isomers as well as a mixture thereof are included within the scope of the present invention.

Concerning the starting thiol compounds (III), (IIIa), (IIIb), (IIIc), (IIId), (IIIe), (IIIf), (IIIg), (IIIh), (IIIi), (IV), (V), and (XXV), there are tautomeric isomers as shown by the following equilibrium:

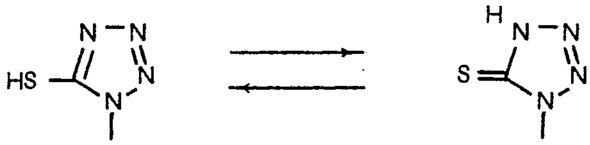

These types of tautomerism between 1-substituted 1H-tetrazole-5-thiol compounds and 1-substituted 4,5-dihydro-1H-tetrazole-5-thione compounds as stated above are well known in the art, and it is obvious to a person skilled in the arts that both of the tautomeric isomers are equilibrated and lie in the reciprocally convertible state, and accordingly it is to be understood that such isomers are included within the same category of the compound *per se*. Accordingly, the both of the tautomeric forms are clearly included within the scope of the present invention. In the present specification, starting compounds including the group of such tautomeric isomers are represented by using one of the expressions therefor, i.e. 1-substituted 1H-tetrazole-5-thiol and the formula:

"

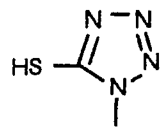

"

Suitable ester moiety for $R^2$, $R^{3a}$ and $R^{3b}$ (esterified carboxy) may be ones such as lower alkyl ester (e.g. methyl ester, ethyl ester, propyl ester, isopropyl ester, butyl ester, isobutyl ester, pentyl ester, hexyl ester, 1-cyclopropylethyl ester, etc.) which may have at least one suitable substituent(s), for example, lower alkanoyloxy(lower)alkyl ester (e.g. acetoxymethyl ester, propionyloxymethyl ester, butyryloxymethyl ester, valeryloxymethyl ester, pivaloyloxymethyl ester, 2-acetoxyethyl ester, 2-propionyloxyethyl ester, hexanoyloxymethyl ester, etc.), lower alkanesulfonyl(lower)alkyl ester (e.g. 2-mesylethyl ester, etc.) or mono(or di or tri)-halo(lower)alkyl ester (e.g. 2-iodoethyl ester, 2,2,2-trichloroethyl ester, etc.); lower alkenyl ester (e.g. vinyl ester, allyl ester, etc.); lower alkynyl ester (e.g. ethynyl ester, propynyl ester, etc.); ar(lower)alkyl ester which may have at least one suitable substituent(s) (e.g. benzyl ester, 4-methoxybenzyl ester, 4-nitrobenzyl ester, phenethyl ester, trityl ester, diphenylmethyl ester, bis(methoxyphenyl)methyl ester, 3,4-dimethoxybenzyl ester, 4-hydroxy-3,5-ditertiarybutylbenzyl ester, etc.); aryl ester which may have at least one suitable substituent(s) (e.g. phenyl ester, 4-chlorophenyl ester, tolyl ester, tertiarybutylphenyl ester, xylyl ester, mesityl ester, cumenyl ester, etc.), and the like.

Preferable examples of the esterified carboxy as mentioned above may include lower alkoxycarbonyl (e.g. methoxycarbonyl, ethoxycarbonyl, propoxycarbonyl, isopropoxycarbonyl, butoxycarbonyl, isobutoxycarbonyl, tert-butoxycarbonyl, pentyloxycarbonyl, tert-pentyloxycarbonyl, hexyloxycarbonyl, 1-cyclopropylethoxycarbonyl, etc.) and phenyl(lower)alkoxycarbonyl (e.g. benzyloxycarbonyl, diphenylmethoxycarbonyl, etc.).

Suitable "$(C_1—C_6)$alkylene" in the terms "hydroxy$(C_1—C_6)$alkylene", "amino$(C_1—C_6)$alkylene", "protected amino$(C_1—C_6)$alkylene", "hydroxyimino$(C_1—C_6)$alkylene wherein the hydrogen atom of the hydroxyimino group may be replaced with a $(C_1—C_6)$ aliphatic hydrocarbon group" for $A_1$, $(C_1—C_6)$alkylene for X and $A_2'$ and $(C_1—C_6)$ alkylene which may have an oxo group for $A_2$ may include straight or branched one and may include methylene, ethylene, trimethylene, propylene, tetramethylene, pentamethylene, hexamethylene and the like.

Suitable "$(C_1—C_6)$aliphatic hydrocarbon group" in the terms "hydroxyimino$(C_1—C_6)$alkylene wherein the hydrogen atom of the hydroxyimino group may be replaced with a $(C_1—C_6)$ aliphatic hydrocarbon group" for $A_1$ and "a $(C_1—C_6)$aliphatic hydrocarbon group" for $R^4$ may include a monovalent radical of a saturated or unsaturated, and straight or branched acyclic aliphatic hydrocarbon, and particularly may include $(C_1—C_6)$alkyl, $(C_2—C_6)$alkenyl, $(C_2—C_6)$alkynyl and the like.

Suitable "lower alkenylene with up to 6C-atoms" for $A_1$, B and B' means straight or branched one and may include $(C_2—C_6)$alkenylene (e.g. vinylene, 1-propenylene, 2-propenylene, 1-methyl-2-propenylene, 1 or 2 or 3-butenylene, 1 or 2 or 3 or 4-pentenylene, 1 or 2 or 3 or 4 or 5-hexenylene, etc.) and $(C_2—C_6)$alkenylidene (e.g. vinylidene, propenylidene, butenylidene, pentenylidene, hexenylidene, etc.).

In this connection, with regard to compounds (III) and (IIIe), it is to be noted that when the group represented by $A_1$ is $(C_2—C_6)$alkenylene, there is a possibility that these compounds exist as geometrical isomers, i.e. cis- and trans-isomers and a mixture thereof. For example, with regard to the object compound (I) wherein the group represented by $—A_1R^{3a}$ is 3-carboxy-1-propenyl, the cis isomer means one geometrical isomer having the partial structure represented by the following formula:

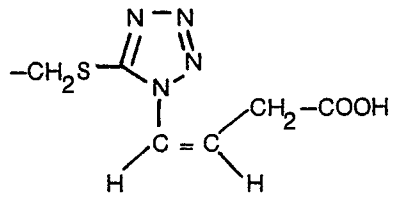

and the trans isomer means the other geometrical isomer having the partial structure represented by the following formula:

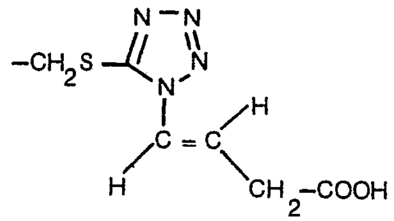

The cis-and trans-isomers as well as a mixture thereof are included within the scope of the present invention.

Suitably "an aliphatic or aromatic group" for $R^{3b}$ may include lower alkyl (e.g. methyl, ethyl, propyl, isopropyl, butyl, isobutyl, tert-butyl, pentyl, neopentyl, tert-pentyl, hexyl, etc.);

lower alkenyl (e.g. vinyl, 1-propenyl, allyl, 1-methylallyl, 1 or 2 or 3-butenyl, 1 or 2 or 3 or 4-pentenyl, 1 or 2 or 3 or 4 or 5-hexenyl, etc.);

aryl (e.g. phenyl, tolyl, xylyl, cumenyl, naphthyl, etc.);

ar(lower)alkyl such as phenyl(lower)alkyl (e.g. benzyl, phenethyl, phenylpropyl, etc.);

hydroxy(lower)alkyl (e.g., hydroxymethyl, hydroxyethyl, hydroxypropyl, hydroxybutyl, etc.); and the like.

Suitable example of Y may include an acid residue (e.g amido, halogen as mentioned above, acyloxy as mentioned above, etc.) and the like.

Suitable "amino having a protective group" for $R^8$ may include phthalimido, succinimido, ethoxycarbonylamino and the like, and preferably phthalimido.

Suitable "($C_1$—$C_6$)alkyl" for $R^9$ means straight or branched one and may include methyl, ethyl, propyl, isopropyl, butyl, isobutyl, tert-butyl, pentyl, hexyl and the like.

Suitable "mercapto-protective group" for $R^{10}$ and $R^{10a}$ may include a conventional protective group such as lower alkyl as mentioned above, ar(lower)alkyl, for example, phenyl(lower)alkyl (e.g., benzyl, phenethyl, phenylpropyl, etc.) and the like.

Suitable "acid residue" for $R^{11}$ and $R^{12}$ may include halogen (e.g., chlorine, bromine, iodine etc.), azide, and the like.

The processes for preparing the new cephem compounds (I) starting from the compounds of the present invention is explained in detail in the following.

The compound (I) or a salt thereof can be prepared by reacting the compound (II) or a salt thereof with the compound (III) or its reactive derivative at the mercapto group or a salt thereof.

Suitable reactive derivative at the mercapto group in the compound (III) may include a metal salt such as an alkali metal salt (e.g. sodium salt, potassium salt, etc.), an alkaline earth metal salt (e.g., magnesium salt, etc.) or the like.

The reaction is usually carried out in a solvent such as water, acetone, chloroform, nitrobenzene, methylene chloride, ethylene chloride, dimethylformamide, methanol, ethanol, ether, tetrahydrofuran or any other conventional solvents which do not adversely influence the reaction, preferably in ones having strong polarity, which may be used as a mixture with water.

When the compound (II) and/or the compound (III) are used in free form in the reactions, the reaction is preferably carried out in the presence of a base such as alkali metal hydroxide, alkali metal carbonate, alkali metal bicarbonate, trialkylamine, pyridine, or a Lewis acid such as boron trifluoride or the like, and preferably carried out around neutral conditions. The reaction temperature is not critical and the reaction is usually carried out at ambient temperature or under warming.

The present invention includes, within its scope, the cases that a protected amino and/or a protected carboxy group are converted into the corresponding free amino and/or the free carboxy group during the reaction or the post-treating step of the present process.

The processes for preparing the compounds (III) of the present invention are explained in detail as follows.

Suitable reactive derivative at the mercapto group of the compounds (V), (IV), (IIIa), (IIIb), (IIIc), (IIId), (IIIe) and (IIIf) can be referred to the ones as exemplified for the compound (III).

Suitable salts of the compounds (IIIh), (VIII), (XIII), (XVI), (XVII), (XIX), (XX), (XXII) and (XXIV) may include an inorganic acid salt (e.g., hydrochloride, hydrobromide, sulfate, etc.), an organic acid salt (e.g., acetate, p-toluenesulfonate, etc.) and the like.

Process A

(V) → (IV)

The compound (IV) or its reactive derivative at the mercapto group or a salt thereof can be prepared by reacting the compound (V) or its reactive derivative at the mercapto group with the compound (VII) or its ester.

Suitable esters of the compound (VII) are referred to the ones exemplified for the ester moiety of the esterified carboxy groups represented by $R^2$.

The present reaction may be carried out in the presence of a base such as alkyl alkali metal (e.g. n-butyllithium, etc.), alkali metal acetate (e.g. sodium acetate, etc.), alkali metal alkoxide (e.g. sodium methoxide, potassium tert-butoxide, etc.), alkali metal hydride (e.g. sodium hydride, etc.), alkali metal hydroxide, alkaline earth metal hydroxide or the like.

The present reaction is usually carried out in a solvent such as tetrahydrofuran or any other solvents which do not adversely affect the reaction.

The reaction temperature is not critical and the reaction is preferably carried out under cooling or at ambient temperature.

Process B

i): optional process

The compound (IV) or its reactive derivative at the mercapto group or a salt thereof wherein $R^3$ is a protected carboxy group may, if desired, be converted into its free form by elimination of the carboxy protective group,

prior to: ii) introduction of the substituent: $=N—OR^4$; or

iii) conversion of the —CO— group to the —CH(OH) group.

In the present elimination reaction, conventional methods used in the elimination reaction of the carboxy protective group, for example, hydrolysis, reduction, elimination using Lewis acid, etc. are applicable. When the carboxy protective group is an ester, it can be eliminated by hydrolysis or elimination using Lewis acid. The hydrolysis is preferably carried out in the presence of a base (e.g. sodium hydroxide, etc.) or an acid (e.g. hydrochloric acid, formic acid, etc.).

The present reaction is usually carried out in a solvent such as alcohol (e.g. methanol, etc.) or any other solvents which do not adversely affect the reaction.

The reaction temperature is not critical and the reaction is preferably carried out under cooling, at ambient temperature or under warming.

ii) (IV) + (VIII) → (IIIa)

The compound (IIIa) or its reactive derivative at the mercapto group or a salt thereof can be prepared by reacting the compound (IV) or its reactive derivative at the mercapto group or a salt thereof with the compound (VIII) or a salt thereof.

The reaction is usually carried out in a conventional solvent such as water, alcohol (e.g., methanol, ethanol, etc.), a mixture thereof or any other ones which do not adversely influence the reaction.

When the compound (VIII) is used in its salt form, the reaction is preferably carried out in the presence of an organic or an inorganic base as exemplified before.

The reaction temperature is not critical, and the reaction is usually carried out from cooling to heating.

iii) (IV) → (IIIb)

i) The compound (IIIb) or its reactive derivative at the mercapto group or a salt thereof can be prepared by reducing the compound (IV) or its reactive derivative at the mercapto group or a salt thereof.

The present reduction can be carried out by a conventional method which is applied to the reduction of the —CO— group to the corresponding —CH(OH)— group, for example, by using a combination of a metal (e.g. zinc, etc.) and an acid (e.g. formic acid, acetic acid, hydrochloric acid, etc.) or a base (e.g. sodium hydroxide, etc.), alkali metal borohydride (e.g. lithium borohydride, etc.), metal amalgam (e.g. aluminum amalgam, etc.) or the like.

The present reaction is preferably carried out in a solvent such as alcohol (e.g. ethanol, etc.) or any other solvents which do not adversely affect the reaction.

The reaction temperature is not critical and the reaction is preferably carried out under cooling, at ambient temperature or under warming.

Process C

i) (IIIa) → (IIId)

The compound (IIId) or its reactive derivative at the mercapto group or a salt thereof can be prepared by reducing the compound (IIIa) or its reactive derivative at the mercapto group or a salt thereof.

The present reduction can be carried out by the methods explained in Process B iii), among which the method of using a combination of a metal (e.g. zinc, etc.) and an acid (e.g. formic acid, hydrochloric acid, etc.) are preferred.

ii) (IIId) → (IIIc)

a) The compound (IIIc) or its reactive derivative at the mercapto group or a salt thereof can be prepared by subjecting the compound (IIId) or its reactive derivative at the mercapto group or a salt thereof to the introduction reaction of the amino group.

The present introduction reaction can be carried out by reacting the compound (IIId) or its reactive derivative at the mercapto group or a salt thereof with an acylating agent. Suitable acylating agent may include a) $R^{13}$—OH (XXVII) (wherein $R^{13}$ is acyl as defined above) or its reactive derivatives or a salt thereof as explained as follows

b) an introducing agent of lower alkoxycarbonyl group, for example, 2-(lower)alkoxycarbonyloxyimino-2-cyanoacetamide (e.g., 2-ethoxycarbonyloxyimino-2-cyanoacetamide, etc.), 2-ethoxycarbonyloxyimino-2-cyanoacetamide, etc.), di(lower)alkyl 2-(lower)alkoxycarbonyloxyiminomalonate (e.g. diethyl 2-tert-butoxycarbonyloxyiminomalonate, etc.), lower alkyl 2-(lower)alkoxycarbonyloxyimino-2-cyanoacetate (e.g. ethyl 2-isobutoxycarbonyloxyimino-2-cyanoacetate, etc.), lower alkyl 2-(lower)alkoxycarbonyloxyiminoacetoacetate (e.g. ethyl 2-tert-butoxycarbonyloxyiminoaceto-acetate, etc.), lower alkoxycarbonyloxyimino-2-phenylacetonitrile (e.g. tert-butoxycarbonyloxyimino-2-phenylacetonitrile, etc.).

The substantially same reaction conditions as set out in Process 2 in EP—B—29998 can be applied in this reaction.

## Process D

(IIIf) → (IIIe)

The compound (IIIe) or its reactive derivative at the mercapto group or a salt thereof can be prepared by subjecting the compound (IIIf) or its reactive derivative at the mercapto group to the reaction to introduce a carboxy group; whereafter, if necessary, the introduction of a carboxy protective group is carried out.

The present reaction can be carried out by a conventional method which is applied for the introduction of a carboxy group to the alkenyl group, for example by reacting the compound (IIIf) with dry ice or carbon dioxide, or the like.

The present reaction is usually carried out in the presence of a base as exemplified in Process A.

The present reaction is usually carried out in a solvent such as tetrahydrofuran, hexan or any other solvents which do not adversely affect the reaction. The reaction temperature is not critical and the reaction is preferably carried out under cooling or at ambient temperature.

## Process E

1) (XIX) + (XVIII) → (XVII)

The compound (XVII) or a salt thereof can be prepared by reacting the compound (XIX) or a salt thereof with the compound (XVIII).

The present reaction may be carried out in the presence of an organic or inorganic base such as alkali metal bicarbonate, alkali metal carbonate, alkaline earth metal bicarbonate, alkaline earth metal carbonate, alkali metal hydroxide, alkaline earth metal hydroxide, tri(lower) alkylamine, N,N-di(lower)alkyl arylamine, pyridine or the like.

The present reaction is usually carried out in a solvent such as acetone, N,N-dimethylformamide or any other solvents which do not adversely affect the reaction.

The reaction temperature is not critical and the reaction is preferably carried out from an ambient temperature to under heating.

2) (XVII) → (XVI)

The compounds (XVI) or a salt thereof can be prepared by subjecting the compound (XVII) or a salt thereof to the elimination of the amino-protective group.

This elimination reaction can be carried out in a similar manner to that of aforementioned Process 3 as described in EP—B—29998.

3) (XVI) → (XV)

The compound (XV) or a salt thereof can be prepared by reacting the compound (XVI) or a salt thereof with carbon disulfide.

The present reaction is usually carried out in a solvent such as alcohol (e.g., methanol, etc.) or any other solvents which do not adversely affect the reaction.

The reaction temperature is not critical and the reaction is preferably carried out under cooling to warming.

4) (XV) + (XIV) → (XIII)

The compound (XIII) or a salt thereof can be prepared by reacting the compound (XV) or a salt thereof with a lower alkylating agent (XIV).

Suitable "lower alkylating agent" may include lower alkyl halide (e.g. methyl iodide, methyl chloride, methyl bromide, ethyl iodide, etc.) and the like.

The present reaction is preferably carried out in a solvent such as alcohol (e.g. ethanol, etc.) or any other solvents which do not adversely affect the reaction.

The reaction temperature is not critical and the reaction is preferably carried out under cooling to warming.

5) (XIII) + (XII) → (IIIg)

The compound (IIIg) or a salt thereof can be preapred by reacting the compound (XIII) or a salt thereof with a hydrazoic acid salt (XII).

Suitable "hydrazoic acid salt" may include an alkali metal azido (e.g. sodium azido, potassium azido, etc.) and the like.

The present reaction is usually carried out in a solvent such as water, alcohol (e.g., methanol, ethanol, etc.) or a mixed solvent thereof or any other solvents which do not adversely affect the reaction.

The reaction temperature is not critical and the reaction is preferably carried out from at ambient to under heating.

Process F

1) (XXIV) + (XXIII) → (XXII)

The compound (XXII) or a salt thereof can be prepared by reacting the compound (XXIV) or a salt thereof with the compound (XXIII).

The present reaction is usually carried out in a solvent such as water, alcohol (e.g., methanol, ethanol, etc.) or a mixed solvent thereof, or any other solvents which do not adversely affect the reaction.

The reaction temperature is not critical and the reaction is preferably carried out under cooling to warming.

2) (XXII) → (XXI)

The compound (XXI) can be prepared by subjecting the compound (XXII) or a salt thereof to the diazotization reaction.

The present diazotization can be carried out by using a conventional diazotizing agent such as nitrous acid, [which may be prepared in situ by reaction of an alkali metal nitrite with an acid (e.g. hydrochloric acid, etc.)], nitrosyl-chloride, lower alkyl nitrite (e.g., t-butyl nitrite, etc.) or the like.

The present reaction may be carried out in the presence of an organic or inorganic acid such as hydrochloric acid, acetic acid or the like.

The present reaction may be carried out in a solvent such as alcohol (e.g., methanol, ethanol, etc.) or any other solvents which do not adversely affect the reaction.

The reaction temperature is not critical and the reaction is preferably carried out under cooling or at ambient temperature.

(3) (XXI) + (XIX) → (XX)

The compound (XX) or a salt thereof can be prepared by reacting the compound (XXI) or its reactive derivative at the hydroxy group with the compound (XIX) or a salt thereof.

Suitable reactive derivative at the hydroxy group of the compound (XXI) may include the compound (XXI) wherein the hydroxy group is transformed into an acid residue such as halogen (e.g., chlorine, bromine, etc.), arenesulfonyloxy (e.g., p-toluenesulfonyloxy, p-nitrobenzenesulfonyl, etc.), haloformyloxy (e.g., chloroformyloxy, etc.) or the like.

The present reaction can be carried out in a similar manner to that of aforementioned Process E-1).

4) (XX) → (IIIg)

The compound (IIIg) or a salt thereof can be prepared by subjecting the compound (XX) or a salt thereof to the elimination reaction of the mercapto-protective group.

The present elimination reaction may be carried out in accordance with a conventional method such as hydrolysis using an organic or inorganic acid (e.g. acetic acid, hydrobromic acid, etc.) or the like.

The present reaction is usually carried out in a solvent such as water or any other solvents which do not adversely affect the reaction.

The reaction temperature is not critical and the reaction is preferably carried out under cooling to heating.

Process G

(XXV) + (XIX) → (IIIi)

The compound (IIIi) or a salt thereof can be prepared by reacting the compound (XXV) or its reactive derivative at the carboxy group or a salt thereof with the compound (XIX) or its reactive derivative at the amino group or a salt thereof.

The present reaction can be carried out in a similar manner to that of aforementioned Process C-ii.

The present invention includes, within its scope, the cases that protected amino and/or esterified carboxy and/or protected hydroxy group(s) are transformed into the corresponding free amino and/or carboxy and/or hydroxy group(s) according to the reaction conditions and kinds of the protective groups in the course of the aforementioned reactions and/or in post-treatment of the reactions in Processes A to G.

In the aforementioned reactions and/or the post-treating of the reactions in Processes A to G of the present invention, the aforementioned geometrical isomer and/or tautomeric isomer may occasionally be transformed into the other geometrical isomer and/or tautomeric isomer and such cases are to be also included in the scope of the present invention.

The compound (III') of the present invention is useful for the preparation of the new cephem compound (I), which exhibits high antimicrobial activity and inhibits the growth of a number of microorganisms including pathogenic Gram-positive and Gram-negative bacteria (see EP—B—29998).

The following preparations and examples are given for the purpose of illustrating the present invention.

Preparation 1

A solution of 1-methyl-1H-tetrazole-5-thiol (23 g) in dry tetrahydrofuran (80 ml) was added over 20 minutes with stirring under dry nitrogen atmosphere to a n-butyllithium (250 ml, 15% in hexane) solution in dry tetrahydrofuran (250 ml) precooled to −15 to −10°C. After stirring at the same temperature for 40 minutes, the mixture was cooled to −60°±5°C and thereto was added dropwise a solution of diethyl oxalate (28.95 g) in dry tetrahydrofuran (80 ml) over 30 min. The reaction mixture was stirred at −60°±5°C for 1.5 hours. 10% aqueous hydrochloric acid (72 ml) was added with stirring and the separated aqueous layer (pH 7.5) was adjusted to pH 2.5 with 10% hydrochloric acid and extracted with ethyl acetate (200 ml × 3). The combined extracts were dried over magnesium sulfate and evaporated to give oily residue of 1-ethoxyalylmethyl-1H-tetrazole-5-thiol (34.3 g).

I.R. (Nujol): 1730 $cm^{-1}$.
N.M.R. ($CDCl_3$, δ): 1.43 (3H, t, J=7Hz), 4.46 (2H, q, J=7Hz), 5.67 (2H, s).

Preparation 2

To a solution of 1-ethoxalylmethyl-1H-tetrazole-5-thiol (18.6 g) in methanol (186 ml) was added 1N aqueous solution of sodium hydroxide (172 ml) at room temperature and the mixture was stirred for 40 minutes. After adjusting to pH 7.5 with 10% hydrochloric acid, the solution was evaporated in vacuo to remove methanol and the remained aqueous solution was washed with ethyl acetate (100 ml). The aqueous solution was adjusted to pH 4.0 and washed again with ethyl acetate to remove impurities. The aqueous solution was adjusted to pH 2.5, and extracted with ethyl acetate, dried over magnesium sulfate and evaporated to give pale yellow powder of 1-oxalomethyl-1H-tetrazole-5-thiol (9.7 g), mp 140 to 150°C (dec.).

I.R. (Nujol): 1750, 1710 $cm^{-1}$.
N.M.R. (DMSO-$d_6$, δ): 5.71 (2H, s).

Preparation 3

A solution of 1-oxalomethyl-1H-tetrazole-5-thiol (10.0 g) and hydroxylamine hydrochloride (4.8 g) in water (100 ml) was adjusted to pH 6.0 with 2N aqueous solution of sodium hydroxide and stirred at room temperature for 3.5 hours. The solution was acidified with 10% hydrochloric acid to pH 2.0, extracted with a mixture of tetrahydrofuran and ethyl acetate (1:1). The extract was washed with a saturated aqueous solution of sodium chloride, dried over magnesium sulfate and evaporated to give 1-(2-carboxy-2-hydroxyiminoethyl)-1H-tetrazole-5-thiol (5.61 g).

N.M.R. (DMSO-$d_6$, δ): 5.19 (2H, s).

Preparation 4

A solution of 1-oxalomethyl-1H-tetrazole-5-thiol (1.09 g) and methoxyamine hydrochloride (0.47 g) in water (15 ml) was adjusted to pH 6.5 with sodium bicarbonate and stirred at room temperature for 2 hours. After washing with ethyl acetate, the aqueous solution was adjusted to pH 1.8 with 10% hydrochloric acid, extracted with ethyl acetate, dried over magnesium sulfate and evaporated to give 1-(2-carboxy-2-methoxyiminoethyl)-1H-tetrazole-5-thiol (1.05 g), mp 84 to 86°C.

I.R. (Nujol): 1685 $cm^{-1}$.
N.M.R. (DMSO-$d_6$, δ): 3.97 (3H, s), 5.17 (2H, s).

Preparation 5

The following compound was prepared according to the similar manners to those of Preparation 1 to 4.
1-(2-Carboxy-2-allyloxyiminoethyl)-1H-tetrazole-5-thiol.

I.R. (Nujol): 1700 $cm^{-1}$.
N.M.R. (DMSO-$d_6$, δ): 4.58 (2H, m), 4.98—5.33 (4H, m), 5.58—6.07 (1H, m).

Preparation 6

1-Oxalomethyl-1H-tetrazole-5-thiol (0.94 g) was dissolved in aqueous 60% ethanol (15 ml) containing sodium hydroxide (1.4 g) and to this solution was added portionwise zinc powder (0.98 g). The mixture was stirred at room temperature for 3.5 hours. After removal of zinc powder by filtration, ethanol was removed under reduced pressure and the remained aqueous solution was adjusted to pH 2.0 with 10% hydrochloric acid. Extraction with ethyl acetate, drying over magnesium sulfate and evaporation gave an oil which was triturated with a mixture of diisopropyl ether and n-hexane (1:1). The crystals were filtered and washed with a mixture of diisopropyl ether and n-hexane (1:1) to give 1-(2-carboxy-2-hydroxyethyl)-1H-tetrazole-5-thiol (0.40 g), mp 143 to 146°C.

I.R. (Nujol): 3320, 3180, 1715 $cm^{-1}$.
N.M.R. ($DMSO-d_6$, δ): 4.17—4.57 (3H, m).

Preparation 7

A mixture of 1-(2-carboxy-2-hydroxyiminoethyl)-1H-tetrazole-5-thiol (19.3 g), 50% formic acid (255 ml), zinc powder (22.3 g) in ethanol (230 ml) was stirred for 5 hours at room temperature. After the addition of trifluoroacetic acid (100 ml), the mixture was stirred for 5 minutes and filtered. The filtrate was evaporated under reduced pressure. The residue was adjusted to pH 13.0 with 4N aqueous solution of sodium hydroxide and then filtered. The filtrate containing disodium 1-(2-carboxylate-2-aminoethyl)-1H-tetrazole-5-thiolate was adjusted to pH 6.0 and thereto were added dioxane (300 ml), 2-tert-butoxycarbonyloxyimino-2-phenylacetonitrile (35.1 g) and triethylamine (26.2 g). The resulting mixture was stirred overnight at room temperature. The reaction mixture was adjusted to pH 7.0 and washed with ethyl acetate. The washed layer was adjusted to pH 2.0 with phosphoric acid and then extracted with ethyl acetate. The extract was washed with a saturated aqueous solution of sodium chloride, dried over magnesium sulfate and evaporated. The residue was pulverized in diisopropyl ether to give 1-(2-carboxy-2-tert-butoxycarboxamidoethyl)-1H-tetrazole-5-thiol (21.2 g).

I.R. (Nujol): 3400, 1718, 1690 $cm^{-1}$.
N.M.R. ($DMSO-d_6$, δ): 1.34 (9H, s), 4.33—4.80 (3H, m), 7.27 (1H, broad s).

Preparation 8

15% solution of n-butyl lithium in hexane (371 ml) was dissolved in dry tetrahydrofuran (150 ml) and cooled to −20°C. To the solution was added dropwise a solution of 1-allyl-1H-tetrazole-5-thiol (38.7 g) in tetrahydrofuran (220 ml) with stirring under a stream of nitrogen gas during a period of 30 minutes. After the addition of solid carbon dioxide, the mixture was stirred for 30 minutes at −10°C. To the mixture was added water (275 ml) and then the resulting mixture was stirred. The aqueous layer was separated adjusted to pH 4.5, washed with ethyl acetate, adjusted to pH 3.8 to 4.0 and extracted with ethyl acetate. The extract was washed with a saturated aqueous solution of sodium chloride, dried over magnesium sulfate and then evaporated.

The residue was washed with diisopropyl ether to give an oil of 1-(3-carboxy-1-propenyl)-1H-tetrazole-5-thiol (5.5 g).

I.R. (Nujol): 1700 $cm^{-1}$.
N.M.R. ($DMSO-d_6$, δ): 3.57 (2H, m), 6.00 (1H, m), 7.05 (1H, m).

The remaining aqueous layer was adjusted to pH 1.5 and extracted with ethyl acetate. The extract was washed with a saturated aqueous solution of sodium chloride, dried over magnesium sulfate and evaporated to give 1-(1-carboxyallyl)-1H-tetrazole-5-thiol (21.3 g).

I.R. (Film): 1710, 1620 $cm^{-1}$.
N.M.R. ($DMSO-d_6$, δ): 5.30—5.70 (2H, m), 5.90—6.67 (2H, m).

Preparation 9

(1) To the solution of N-(3-bromopropyl) phthalimide (402 g) in acetone (2.5 l) were added 1-methylpiperazine (225 g) and potassium carbonate (415 g). The resulting mixture was refluxed with stirrring for 3.5 hours. The reaction mixture was cooled and then filtered. The filter cake was washed with acetone (500 ml). The filtrate and washing were combined and evaporated under reduced pressure. The remaining starting materials in the residual oil were azeotropically removed with benzene to give an oil of N-[3-(4-methyl-1-piperazinyl)propyl]phthalimide (502 g).

I.R. (Film): 1770, 1700 $cm^{-1}$.

(2) To a solution of N-[3-(4-methyl-1-piperazinyl)propyl]phthalimide (502 g) in ethanol (3.0 l) was added 100% hydrazine hydrate (187.6 g). The resulting mixture was refluxed with stiving for 1.5 hours. The

reaction mixture was cooled and then filtered. The filter cake was washed with ethanol (1 l). The filtrate and washing were combined and evaporated, under reduced pressure. The residual oil was distilled under reduced pressure to give 3-(4-methyl-1-piperazinyl)propylamine (146.6 g), bp 34 mmHg/127 to 128°C.

(3) To a solution of potassium hydroxide (57.3 g) in methanol (250 ml) was added 3-(4-methyl-1-piperazinyl)propylamine (146 g) and thereto was added carbon disulfide (70.6 g) with stirring under ice cooling over a period of 40 minutes. The resulting mixture was stirred for 3.5 hours under ice cooling. The reaction mixture was evaporated under reduced pressure. The residual oil was dissolved in water (400 ml) and washed with diethyl ether twice. The washed aqueous layer was ice-cooled and thereto was added methyl iodide (132.1 g) with stirring. The resulting mixture was stirred for 2 hours under ice-cooling, and extracted with ethyl acetate (400 ml × 3) and chloroform (400 ml × 2). The extracts were combined, dried over magnesium sulfate and then evaporated under reduced pressure to give methyl N-[3-(4-methyl-1-piperazinyl)propyl]dithiocarbamate (139.3 g).

Thus obtained product was used directly in the next step reaction without further purification.

(4) To a solution of methyl N-[3-(4-methyl-1-piperazinyl)propyl dithiocarbamate obtained in Preparation 1 (3) (139.3 g) in a mixture of water (420 ml) and ethanol (280 ml) was added sodium azide (47.6 g). The resulting mixture was refluxed with stirring for 3 hours. The reaction mixture was concentrated under reduced pressure. The concentrate was washed successively with ethyl acetate and diethyl ether and then evaporated. To the residue was added ethanol and the mixture was filtered. The filtrate was evaporated under reduced pressure, and to the residual oil was added 6N hydrochloric acid. The mixture was evaporated under reduced pressure. The residue was recrystallized from isopropyl alcohol containing water to give 1-[3-(4-methyl-1-piperazinyl)propyl]-1H-tetrazole-5-thiol dihydrochloride (48.1 g), mp 239 to 243°C.

N.M.R. ($D_2O$, δ): 2.3—2.8 (2H, m), 3.07 (3H, s) 3.3—3.7 (2H, m), 3.75 (8H, s), 4.42 (2H, t,J=6Hz).

Preparation 10

(1) To the solution of N-(3-bromopropyl)phthalimide (20.1 g) in acetone (225 ml) were added.

1-benzyl piperazine (19.8 g) and potassium carbonate (31.1 g). The resulting mixture was refluxed with stirring for 3 hours. The reaction mixture was cooled and then filtered. The filter cake was washed with acetone. The filtrate and washing were combined and evaporated under reduced pressure to give the oily crude product of the object compound (36.2 g) This oily product was subjected to column chromatography on silica gel. The fractions containing the object compound were collected to give an oil of N-[3-(4-benzyl-1-piperazinyl)propyl]phthalimide (22.4 g).

(2) To a solution of N-[3-(4-benzyl-1-piperazinyl)propyl]phthalimide (22.4 g) in ethanol (250 m) was added 100% hydrazine hydrate (8.02 g). The resulting mixture was refluxed with stirring for 2.5 hours. The reaction mixture was cooled and then filtered. The filter cake was washed with ethanol. The filtrate and washing were combined and evaporated under reduced pressure. To the residue was added 5% aqueous solution of potassium hydroxide and the mixture was extracted with chloroform. The extract was washed with water, dried over magnesium sulfate and then evaporated under reduced pressure to give an oil of 3-(4-benzyl-1-piperazinyl)propylamine (12.4 g).

N.M.R. ($CDCl_3$, S): 1.4 (2H, s), 1.6 (2H, tt, J=7Hz), 2.4 (2H, t, J=7Hz), 2.47 (8H, s), 2.73 (2H, t, J=7Hz), 3.5 (2H, s), 7.27 (5H, s).

(3) To a solution of potassium hydroxide (1.3 g) in methanol (9 ml) was added 3-(4-benzyl-1-piperazinyl)propylamine (4.9 g) and thereto was added carbon disulfide (1.8 g) under ice cooling over a period of 10 minutes. The resulting mixture was stirred for 4 hours under ice cooling. The reaction mixture was evaporated under reduced pressure. The residual oil was dissolved in water (20 ml) and washed with diethyl ether. The washed aqueous layer was ice-cooled and thereto was added methyl iodide (3.0 g) with stirring under ice-cooling over a period of 10 minutes. The resulting mixture was stirred for 4 hours under ice-cooling. The reaction mixture was extracted with ethyl acetate, dried over magnesium sulfate and then evaporated under reduced pressure to give an oil of methyl N-[3-(4-benzyl-1-piperazinyl) propyl]dithiocarbamate (4.6 g).

(4) To a solution of methyl N-[3-(4-benzyl-1-piperazinyl)propyl]dithiocarbamate (4.6 g) in a mixture of water (12 ml) and ethanol (8 ml) was added sodium azide (1.2 g). The resulting mixture was refluxed with stirring for 3 hours. The reaction mixture was concentrated under reduced pressure. The concentrate was washed successively with ethyl acetate and diethyl ether and then evaporated. To the residue was added ethanol and the mixture was filtered. The filtrate was evaporated under reduced pressure. To the residue was added 6N hydrochloric acid. The mixture was evaporated under reduced pressure and the residue was recrystallized from isopropyl alcohol containing water to give 1-[3-(4-benzyl-1-piperazinyl)propyl]-1H-tetrazole-5-thiol dihydrochloride (2.5 g), mp 234 to 237°C.

N.M.R. (DMSO-$d_6$, δ): 2.1—3.0 (2H, m), 3.1—4.1 (10H, m), 4.42 (2H, s), 4.4 (2H, t), 7.3—8.0 (5H, m).

Preparation 11

(1) To the soluton of N-(3-bromopropyl)phthalimide (38.8 g) in acetone (400 ml) were added.

1-ally-piperazine (18.5 g) and potassium carbonate (60.8 g). The resulting mixture was refluxed with stirring for 5 hours. The reaction mixture was cooled and then filtered. The filter cake was washed with acetone. The filtrate and washing were combined and evaporated under reduced pressure. The residual oil was subjected to column chromatography on silica gel. The fractions containing the object compound were collected and then evaporated under reduced pressure to give an oil of N-[3-(4-allyl-1-piperazinyl)propyl]phthalimide (35.6 g).

(2) To a solution of N-[3-(4-allyl-1-piperazinyl)propyl]phthalimide (35.6 g) in ethanol (400 ml) was added 100% hydrazine hydrate (14.3 g). The resulting mixture was refluxed with stirring for 2.5 hours. The reaction mixture was cooled and then filtered. The filter cake was washed with ethanol. The filtrate and washing were combined and evaporated under reduced pressure. The residual oil was subjected to column chromatography (aluminum oxide), eluting with a mixture of chloroform and methanol (10:1). The fractions containing the object compound were collected and then evaporated under reduced pressure to give an oil of 3-(4-allyl-1-piperazinyl)propylamine (18.8 g).

N.M.P. ($CCl_4$, δ): 1.43 (2H, s), 1.52 (2H, tt, J=7Hz), 2.4 (8H, broad s), 2.33 (2H, t, J=7Hz), 2.67 (2H, t, J=7Hz), 2.92 (2H, d, J=6Hz), 5.0—6.0 (3H, m).

(3) To a solution of potassium hydroxide (6.3 g) in methanol (40 m) was added 3-(4-allyl-1-piperazinyl)propylamine (18.8 g) and thereto was added carbon disulfide (7.8 g) under ice cooling over a period of 10 minutes. The resulting mixture was stirred for 2 hours under ice cooling. The reaction mixture was evaporated under reduced pressure.

The residual oil was dissolved in water and washed with diethyl ether. The washed aqueous layer was ice-cooled and thereto was added methyl iodide (14.6 g) with stirring. The resulting mixture was stirred for 2 hours under ice-cooling. The reaction mixture was extracted with ethyl acetate, the extract was washed with a saturated aqueous solution of sodium chloride, dried over magnesium sulfate and then evaporated under reduced pressure to give an oil of methyl N-[3-(4-allyl-1-piperazinyl)propyl]-dithiocarbamate (17.7 g).

(4) To a solution of methyl N-[3-(4-allyl-1-piperazinyl)propyl]dithiocarbamate (17.7 g) in a mixture of water (60 ml) and ethanol (40 ml) was added sodium azide (5.5 g). The resulting mixture was refluxed with stirring for 3 hours. The reaction mixture was concentrated under reduced pressure. The concentrate was washed successively with ethyl acetate and diethyl ether and then evaporated. To the residue was added ethanol and the mixture was filtered. The filtrate was evaporated under reduced pressure. To the residue was added 6N hydrochloric acid. The mixture was evaporated under reduced pressure. The residue was recrystallized from methanol containing water to give 1-[3-(4-allyl-1-piperazinyl)propyl]-1H-tetrazole-5-thiol dihydrochloride (2.5 g), mp 211 to 215°C (dec.).

N.M.R. ($D_2O$, δ): 2.2—2.8 (2H, m), 3.78 (8H, s), 3.67 (2H, t, J=6Hz), 3.97 (2H, d, J=7Hz), 4.50 (2H, t, J=7Hz), 5.5—6.5 (3H, m).

Preparation 12

(1) To the solution of N-(3-bromopropyl)phthalimide (40.5 g) in acetone (375 ml) were added 1-(2-hydroxyethyl)piperazine (31.5 g) and potassium carbonate (63.0 g). The resulting mixture was refluxed with stirring for 3 hours. The reaction mixture was filtered and the filtrate was evaporated. The residual oil was subjected to column chromatography on silica gel, eluting with a mixture of chloroform and methanol (9:1). The fractions containing the object compound were collected and then evaporated to give an oil of N-[3-[4-(2-hydroxyethyl)-1-piperazinyl]propyl]phthalimide (25.5 g).

(2) To a solution of N-[3-[4-(2-hydroxyethyl)-1-piperazinyl]propyl]phthalimide (14.4 g) in ethanol (145 ml) was added 100% hydrazine hydrate (6.6 g). The resulting mixture was refluxed with stirring for 2 hours. The reaction mixture was cooled and then filtered. The filter cake was washed with ethanol. The filtrate and washing were combined and evaporated under reduced pressure. The residual oil was subjected to column chromatography (Aluminum oxide), eluting with a mixture of chloroform and methanol (20:1). The fractions containing the object compound were collected and then evaporated to give an oil of 3-[4-(2-hydroxyethyl)-1-piperazinyl)]propylamine (5.0 g).

N.M.R. (DMSO-$d_6$, S) 1.48 (2H, tt, J=7Hz), 2.28 (2H, t, J=7Hz), 2.53 (2H, t, J=7Hz), 2.1—2.7 (10H, m), 3.03 (3H, s), 3.16 (2H, t, J=7Hz).

(3) To a solution of potassium hydroxide (1.5 g) in methanol (11 ml) was added 3-[4-(2-hydroxyethyl)-1-piperazinyl]propylamine (5.0 g) and thereto was added carbon disulfide (2.0 g) under ice cooling over a period of 10 minutes. The resulting mixture was stirred for 2 hours under ice cooling. The reaction mixture was evaporated under reduced pressure. The residual oil was dissolved in water (10 ml), washed with diethyl ether. The washed aqueous layer was ice-cooled and thereto was added methyl iodide (3.8 g) with stirring. The resulting mixture was stirred for 2 hours under ice-cooling, extracted with ethyl acetate, dried over magnesium sulfate and then evaporated to give methyl N-[3-[4-(2-hydroxy-ethyl)-1-piperazinyl]propyl]-dithio-carbamate (4.8 g).

(4) To a solution of methyl N-[3-[4-(2-hydroxy-ethyl)-1-piperazinyl]-propyl]-dithiocarbamate (4.8 g) in a mixture of water (15 ml) and ethanol (10 ml) was added sodium azide (1.5 g). The resulting mixture was refluxed with stirring for 4 hours. The reaction mixture was concentrated under reduced pressure. The concentrate was dissolved in water and the solution was washed successively with ethyl acetate and diethyl ether and then evaporated. To the residue was added ethanol and the mixture was filtered. The filtrate was evaporated under reduced pressure. The residue was recrystallized from isopropyl alcohol to give 1-[3-[4-(2-hydroxy-ethyl)-1-piperazinyl]propyl]-1H-tetrazole-5-thiol (1.35 g).

N.M.R. ($D_2O$, δ): 2.0—3.0 (14H, m), 3.79 (2H, t, J=7Hz), 4.40 (2H, t, J=7Hz).

Preparation 13

(1) To the solution of N-(3-bromopropyl)phthalimide (53.0 g) in acetone (400 ml) were added.

1-ethoxycarbonyl piperazine (50.0 g) and potassium carbonate (82.0 g). The resulting mixture was refluxed with stirring for 15 hours. The reaction mixture was cooled and then filtered. The filter cake was washed with acetone. The filtrate and washing were combined and evaporated under reduced pressure. The residue was recrystallized from a mixture of benzene and n-hexane to give N-[3-(4-ethoxycarbonyl-1-piperazinyl)propyl]phthalimide (65.2 g), mp 77 to 84°C.

(2) To a solution of N-[3-(4-ethoxycarbonyl-1-piperazinyl)propyl]phthalimide (28.9 g) in ethanol (300 ml) was added 100% hydrazine hydrate (8.4 g). The resulting mixture was refluxed with stirring for 4 hours. The reaction mixture was cooled and then filtered. The filter cake was washed with ethanol. The filtrate and washing were combined and evaporated under reduced pressure. After the addition of an aqueous solution of sodium hydroxide to the residue, the mixture was extracted with chloroform. The extract was washed with water, dried over magnesium sulfate and then evaporated under reduced pressure to give an oil of 3-(4-ethoxycarbonyl-1-piperazinyl)propylamine (10.9 g).

N.M.R. ($CDCl_3$, δ): 1.27 (3H, t, J=7Hz), 1.63 (2H, tt, J=7Hz), 2.3—2.6 (6H, m), 2.80 (2H, t, J=7Hz), 3.4—3.7 (4H, m), 4.17 (2H, q, J=7Hz).

(3) To a solution of potassium hydroxide (2.9 g) in methanol (40 m) was added 3-(4-ethoxycarbonyl-1-piperazinyl)propylamine (10.9 g) and thereto was added carbon disulfide (3.9 g) under ice cooling over a period of 5 minutes. The resulting mixture was stirred for 4 hours under ice cooling.

The reaction mixture was evaporated under reduced pressure. The residual oil was dissolved in water (40 ml) and washed with diethyl ether. The washed aqueous layer was ice-cooled and thereto was added methyl iodide (7.2 g) with stirring. The resulting mixture was stirred for 2 hours under ice-cooling, and extracted with ethyl acetate. The extract was dried over magnesium sulfate and then evaporated under reduced pressure to give an oil of methyl N-[3-(4-ethoxy-carbonyl-1-piperazinyl)propyl]dithiocarbamate (11.7 g).

(4) To a solution of methyl N-[3-(4-ethoxy-carbonyl-1-piperazinyl)propyl]dithiocarbamate (11.7 g) in a mixture of water (40 ml) and ethanol (30 ml) was added sodium azide (3.3 g). The resulting mixture was refluxed with stirring for 4 hours. The reaction mixture was concentrated under reduced pressure. After the addition of water to the residue, the mixture was washed with diethyl ether and then evaporated. To the residue was added ethanol and the mixture was filtered. The filtrate was evaporated under reduced pressure. After the addition of a mixture of hydrochloric acid and ethanol to the residue, the resulting mixture was evaporated under reduced pressure. The residue was triturated with acetone to give crude crystals of the object compound (5.7 g), which were recrystallized from ethanol to give 1-[3-(4-ethoxycarbonyl-1-piperazinyl)propyl]-1H-tetrazole-5-thiol hydrochloride (2.1 g), mp 192 to 197°C (dec.).

N.M.R. (DMSO-$d_6$, δ): 1.22 (3H, t, J=7Hz), 2.1—2.5 (2H, m), 3.0—3.5 (6H, m), 3.5—3.9 (4 H, m), 4.13 (2H, q, J=7Hz), 4.37 (2H, t, J=7Hz).

Preparation 14

(1) To the solution of N-(3-bromopropyl)phthalimide (30.1 g) in acetone (200 ml) were added 1-isopropyl piperazine (23.0 g) and potassium carbonate (41.4 g). The resulting mixture was refluxed with stirring for 4 hours. The reaction mixture was cooled and then filtered. The filter cake was washed with acetone. The fitrate and washing were combined and evaporated under reduced pressure to give an oil of N-[3-(4-isopropyl-1-piperazinyl)propyl]phthalimide (43.5 g).

(2) To a solution of N-[3-(4-isopropyl-1-piperazinyl)propyl]phthalimide (43.1 g) in ethanol (350 ml) was added 100% hydrazine hydrate (11.2 g). The resulting mixture was refluxed with stirring for 3 hours. The reaction mixture was cooled and then filtered. The filter cake was washed with ethanol. The filtrate and washing were combined and concentrated under reduced pressure. The residual oil was subjected to column chromatography (aluminum oxide), eluting with a mixture of chloroform and methanol (20:1). The fractions containing the object compound were collected and then evaporated under reduced pressure. The residual oil was distilled under reduced pressure to give 3-(4-isopropyl-1-piperazinyl)propylamine (15.0 g), (bp 45 mmHg/150°C).

(3) To a solution of potassium hydroxide (4.9 g) in methanol (40 ml) was added 3-(4-isopropyl-1-piperazinyl)propylamine (14.7 g) and thereto was added carbon disulfide (6.0 g) under ice cooling over a period of 20 minutes. The resulting mixture was stirred for 3.5 hours under ice cooling. The reaction mixture was evaporated under reduced pressure. The residual oil was dissolved in water (40 ml) and washed with diethyl ether. The washed aqueous layer was ice-cooled and thereto was added methyl iodide (11.3 g) with stirring over a period of about 5 minutes.

The resulting mixture was stirred for 2 hours under ice-cooling extracted with chloroform. The extract was washed with water, dried over magnesium sulfate and then evaporated to give an oil of methyl N-[3-(4-isopropyl-1-piperazinyl)propyl]dithiocarbamate (15.6 g).

(4) To a solution of methyl N-[3-(4-isopropyl-1-piperazinyl)propyl]dithiocarbamate (15.1 g) in a mixture of water (50 ml) and ethanol (40 ml) was added sodium azide (4.6 g). The resulting mixture was refluxed with stirring for 4.5 hours. The reaction mixture was concentrated under reduced pressure. The concentrate was washed successively with diethyl ether and ethyl acetate and then evaporated. To the residue was added ethanol and the mixture was filtered. The filtrate was evaporated under reduced pressure. After the addition of a little amount of mixture of hydrochloric acid and ethanol to the residual oil, the product was recrystallized from methanol to give 1-[3-(4-isopropyl-1-piperazinyl)propyl]-1H-tetrazole-5-thiol dihydrochloride (3.6 g).

N.M.R. (DMSO-$d_6$, δ): 1.28 (6H, d, J=6Hz), 2.1—2.5 (2H, m), 3.1—3.8 (3H, m), 3.58 (8H, s), 4.33 (2H, t, J=8Hz).

## Preparation 15

(1) To the solution of N-(3-bromopropyl)phthalimide (15.6 g) in acetone (150 ml) were added 1-phenylpiperazine (10.4 g) and potassium carbonate (12.0 g). The resulting mixture was refluxed with stirring for 4.5 hours. The reaction mixture was cooled and then filtered. The filter cake was washed with acetone. The filtrate and washing were combined and evaporated under reduced pressure. The residue was recrystallized from ethanol to give an oil of N-[3-(4-phenyl-1-piperazinyl)propyl]phthalimide (18.3 g), mp 129 to 134°C.

(2) To a solution of N-[3-(4-phenyl-1-piperazinyl)propyl]phthalimide (18.3 g) in ethanol (300 ml) was added 100% hydrazine hydrate (5.3 g). The resulting mixture was refluxed with stirring for 2 hours. The reaction mixture was evaporated under reduced pressure. After the addition of dil. aqueous solution of sodium hydroxide to the residue, the mixture was extracted with chloroform. The extract was washed with water, dried over magnesium sulfate and then evaporated under reduced pressure to give 3-(4-phenyl-1-piperazinyl)propylamine (11.2 g).

N.M.R. ($CCl_4$ δ): 1.47 (2H, broad s), 1.57 (2H, tt, J=7Hz), 2.38 (2H, t, J=7Hz), 2.72 (2H, t, J=7Hz), 2.4—2.7 (4H, m), 3.0—3.2 (4H, m), 6.6—7.4 (5H, m).

(3) To a solution of potassium hydroxide (2.85 g) in methanol (25 ml) was added 3-(4-phenyl-1-piperazinyl)propylamine (11.1 g) and thereto was added carbon disulfide (3.85 g) under ice cooling over a period of 10 minutes. The resulting mixture was stirred for 3 hours under ice cooling. The reaction mixture was evaporated under reduced pressure. The residual oil was dissolved in water (40 ml) and washed with diethyl ether. The washed aqueous layer was ice-cooled and thereto was added methyl iodide (7.2 g) with stirring. The resulting mixture was stirred for 2 hours under ice-cooling and extracted with ethyl acetate. The extract was washed with water, dried over magnesium sulfate and then evaporated to give methyl N-[3-(4-phenyl-1-piperazinyl)propyl]dithiocarbamate (12.9 g), mp 75 to 79°C.

(4) To a solution of methyl N-[3-(4-phenyl-1-piperazinyl)propyl]dithiocarbamate (12.9 g) in a mixture of water (20 m) and ethanol (30 m) was added sodium azide (3.6 g). The resulting mixture was refluxed with stirring for 4.5 hours. The reaction mixture was concentrated under reduced pressure. After the addition of water to the residue, the mixture was washed successively with ethyl acetate and diethyl ether and then evaporated. To the residue was added ethanol (100 m) and the mixture was filtered. The filtrate was evaporated under reduced pressure. The residue was recrystallized from isopropyl alcohol twice to give 1-[3-(4-phenyl-1-piperazinyl)propyl]-4,5-dihydro-1H-tetrazole-5-thione (3.2 g), mp. 200 to 205°C.

I.R. (Nujol): 3270, 1600 $cm^{-1}$.

N.M.R. (DMSO-$d_6$, δ): 1.8—2.1 (2H, m), 3.0—3.2 (4H, m), 4.17 (2H, t, J=6Hz), 6.8—7.5 (5H, m).

A solution of the above obtained product in a little amount of water was acidified with dil. hydrochloric acid and then adjusted to pH 7.0 with an aqueous solution of sodium bicarbonate. The precipitates were collected by filtration, washed with water and dried to give 1-[3-(4-phenyl-1-piperazinyl)propyl]-1H-tetrazole-5-thiol, mp 220 to 233°C (dec.).

I.R. (Nujol): 2350, 1590 $cm^{-1}$.

N.M.R. (DMSO-$d_6$, δ): 2.0—2.3 (2H, m), 4.3 (2H, t, J=6Hz), 6.8—7.5 (5H, m).

## Preparation 16

The following compounds were prepared according to the similar manners to those of Preparation 9—15.

(1) 1-[2-(4-Methyl-1-piperazinyl)ethyl]-1H-tetrazole-5-thiol hydrobromide, mp 216 to 222°C.

N.M.R. ($D_2O$, δ) 2.80 (3H, s), 2.80 (4H, m), 3.04 (2H, t, J=6Hz), 3.28 (4H, m), 4.48 (2H, t, J=6Hz).

(2) 1-(4-Methyl-1-piperazinyl)carbonylmethyl)-1H-tetrazole-5-thiol, mp > 250°C.

| Elemental Analysis: | C | H | N |
|---|---|---|---|
| Calcd | 39.65 | 5.82 | 34.69 |
| Found | 39.33 | 5.68 | 34.52 |

Preparation 17

(1) A mixture of 4-(2-hydroxyethyl) thosemicarbazide (10.3 g), benzyl chloride (10.6 g), water (30 ml) and methanol (60 ml) was stirred for 20 hours at room temperature to give a homogeneous solution containing benzyl N-(2-hydroxyethyl)thiocarbazimidate. The solution was ice-cooled and thereto was little by little added sodium nitrite (5.24 g) with stirring and then dropwise added conc. hydrochloric acid (3.5 ml) over a period of 30 minutes. The resulting mixture was stirred for an hour under ice-cooling. To the reaction mixture were added potassium carbonate (18 g) and water (30 ml). The mixture was stirred and then concentrated under reduced pressure. The concentrate was extracted with chloroform and the extract was washed with water, dried over magnesium sulfate and then evaporated under reduced pressure. The residual oil was subjected to column chromatography on silica gel. The fractions containing the object compound were collected to give an oil of 1-(2-hydroxyethyl)-5-benzylthio-1H-tetrazole (12.1 g).

I.R. (Film): 3380, 1595, 1490 $cm^{-1}$.
N.M.R. ($CDCl_3$, δ): 3.1 (1H, m), 4.1 (2H, m), 4.23 (2H, m), 4.50 (2H, s), 7.33 (5H, s).

(2) To a solution of 1-(2-hydroxyethyl)-5-benzylthio-1H-tetrazole (7.0 g) in pyridine (50 ml) was added p-toluenesulfonyl chloride (6.3 g) and the mixture was stirred for 3 hours. After the addition of water, the reaction mixture was extracted with ethyl acetate. The extract was washed with water, dried over magnesium sulfate and then concentrated under reduced pressure. To the residual oil containing 1-(2-tosyloxyethyl)-5-benzylthio-1H-tetrazole were added 1-methylpiperazine (12.0 g), potassium carbonate (12.2 g) and N,N-dimethylformamide (55 m) and the mixture was stirred for 5 hours at 50°C. After the addition of water, the reaction mixture was extracted with ethyl acetate. The extract was washed with water, dried over magnesium sulfate and evaporated under reduced pressure. The crude product was subjected to column chromatography on silica gel. The fractions containing the object compound was collected to give an oil of 1-[2-(4-methyl-1-piperazinyl)ethyl]-5-benzylthio-1H-tetrazole (6.5 g).

I.R. (Film): 1600, 1498, 1460, 1164 $cm^{-1}$.
N.M.R. ($CDCl_3$, δ): 2.23 (3H, s), 2.1—2.7 (8H, m), 2.7 (2H, t, J=6.0Hz), 4.17 (2H, t, J=6.0Hz), 4.5 (2H, s), 7.27 (5H, s).

(3) A mixture of 1-[2-(4-methyl-1-piperazinyl)ethyl]-5-benzylthio-1H-tetrazole (5.4 g), acetic acid (40 ml) and conc. hydrobromic acid (40 ml) was heated at 70 to 80°C for 8 hours. After the concentration of the reaction mixture, the concentrate was diluted with water. The mixture was washed with diethyl ether twice and evaporated under reduced pressure. The residue was recrystallized from methanol. A solution of the obtained crystals in a little amount of water was adjusted to pH 6.0 with 1N aqueous solution of potassium hydroxide, washed with ethyl acetate and then evaporated under reduced pressure. To the residue was added hot ethanol and insoluble materials were filtered out. The filtrate was cooled and the precipitated crystals were collected by filtration to give 1-[2-(4-methyl-1-piperazinyl)ethyl]-1H-tetrazole-5-thiol hydrobromide (1.1 g), mp 216 to 222°C.

N.M.R. ($D_2O$, δ): 2.80 (3H, s), 280 (4H. m), 3.04 (2H, t, J=6Hz), 3.28 (4H, m), 4.48 (2H, t, J=6Hz).

Preparation 18

The following compounds were prepared according to the similar manner to that of Preparation 17.
(1) 1-[3-(4-Methyl-1-piperazinyl)propyl]-1H-tetrazole-5-thiol·dihydrochloride, mp 239 to 243°C.
(2) 1-[3-(4-Benzyl-1-piperazinyl)propyl]-1H-tetrazole-5-thiol·dihydrochloride, mp 234 to 237°C.
(3) 1-[3-(4-Allyl-1-piperazinyl)propyl]-1H-tetrazole-5-thiol·dihydrochloride, mp 211 to 215°C (dec.).
(4) 1-[3-[4-(2-hydroxyethyl)-1-piperazinyl]propyl]-1H-tetrazole-5-thiol.

N.M.R. ($D_2O$, δ): 2.0—3.0 (14H, m), 3.79 (2H, t, J=7Hz), 4.40 (2H, t, J=7Hz).

(5) 1-[3-(4-Ethoxycarbonyl-1-piperazinyl)propyl]-1H-tetrazole-5-thiol, mp 192 to 197°C (dec.).
(6) 1-[3-(4-Isopropyl-1-piperazinyl)propyl]-1H-tetrazole-5-thiol·dihydrochloride.

N.M.R. (DMSO-$d_6$): 1.28 (6H, d, J=6Hz), 2.1—2.5 (2H, m), 3.1—3.8 (3H, m), 3.58 (8H, s), 4.33 (2H, t, J=8Hz).

(7) 1-[3-(4-Phenyl-1-piperazinyl)propyl]-1H-tetrazole-5-thiol, mp 220 to 223°C (dec.).
(8) 1-(4-Methyl-1-piperazinyl)carbonylmethyl-1H-tetrazole-5-thiol, mp > 250°C.

Preparation 19

To a solution of 1-carboxymethyl-1H-tetrazole-5-thiol (3.2 g) in dry acetone (60 ml) was added triethyl amine (4.2 g). After the addition of a solution of pivaloyl chloride (4.8 g) in dry acetone (10 ml) with stirring at −15°C over a period of 5 minutes, the mixture was stirred for one hour and 15 minutes at the same temperature. A solution of 1-methylpiperazine (4.0 g) in dry acetone (10 ml) was dropwise added over a period of 5 minutes and the resulting mixture was stirred at −15°C for 40 minutes and under ice-cooling for a further 2.5 hours. After the evaporation of the reaction mixture under reduced pressure, water was added to the residue, 4N aqueous solution of sodium hydroxide was added until the crystals were dissolved. The resulting solution was evaporated under reduced pressure and the residue was crystallized from water to give 1-(4-methyl-1-piperazinyl)carbonylmethyl-1H-tetrazole-5-thiol (2.2 g). mp > 250°C.

| Elemental Analysis: | C | H | N |
|---|---|---|---|
| Calcd. | 39.65 | 5.82 | 34.69 |
| Found. | 39.33 | 5.68 | 34.52 |

Example 1

To a suspension of 7-aminocephalosporanic acid (0.99 g) and 1-(2-carboxy-2-methoxyiminoethyl)-1H-tetrazole-5-thiol (0.95 g) in dry acetonitrile (10 ml) was added boron trifluoride etherate (1.55 g) and the resulting solution was stirred at 50°C for 2 hours. Water (10 ml) was added to the reaction mixture and the solution was adjusted to pH 3.5 with conc. aqueous ammonia under ice-cooling to give precipitates which were filtered, washed with water and dried to give 7-amino-3-[1-(2-carboxy-2-methoxyiminoethyl)-1H-tetrazol-5-yl]thiomethyl-3-cephem-4-carboxylic acid (1.0 g), mp 170 to 180°C (dec.).

I.R. (Nujol): 1800, 1705, 1615 $cm^{-1}$.

N.M.R. (DMSO-$d_6$, δ): 3.63 (2H, ABq, J=18Hz), 3.96 (3H, s), 4.30 (2H, ABq, J=14Hz), 4.78 (1H, d, J=5Hz), 4.97 (1H, d, J=5Hz), 5.27 (2H, s).

Example 2

To a suspension of 7-[2-methoxyimino-2-(2-aminothiazol-4-yl)acetamido]cephalosporanic acid (syn isomer) (1.14 g) and 1-(2-carboxy-2-methoxyiminoethyl)-1H-tetrazole-5-thiol (0.81 g) in water (20 ml) was added sodium bicarbonate and the solution was adjusted to pH 6.5 and then stirred for 5 hours at 60°C. After cooling at 10 to 15°C, the solution was adjusted to pH 2.5 with 10% hydrochloric acid. The precipitates were collected by filtration, washed with water and then dried to give 7-[2-methoxyimino-2-(2-aminothiazol - 4 - yl)acetamido] - 3 - [1 - (2 - carboxy - 2 - methoxyiminoethyl) - 1H - tetrazol - 5 - yl]thiomethyl - 3 - cephem - 4 - carboxylic acid (syn isomer: in the 7-position).

I.R. (Nujol): 3270, 1770, 1645 $cm^{-1}$.

Example 3

A solution of 7-[2-methoxyimino-2-(2-aminothiazol-4-yl)acetamido]cephalosporanic acid (syn isomer) (20.0 g) and 1-[3-(4-methyl-1-piperazinyl)propyl]-1H-tetrazole-5-thiol·dihydrochloride (16.6 g) in water (1 l) was adjusted to pH 6.0 with a saturated aqueous solution of sodium bicarbonate. The mixture was stirred for 3 hours and 20 minutes at 63 to 65°C while the pH was maintained at 6.0 to 6.2 with 5% hydrochloric acid. The reaction mixture was ice-cooled and adjusted to pH 7.3 with a saturated aqueous solution of sodium bicarbonate and thereto was added cold water (3 l). The resulting solution was subjected to column chromatograph (CM-Cephadex, H-type: 1 l), eluting with water (200 ml), 1% (2 l), 2% (2 l) and 4% (4 l) aqueous solution of sodium chloride. The fractions containing the object compound were collected. The resulting mixture (about 2 l) was adjusted to pH 4.5 with a saturated aqueous solution of sodium bicarbonate and subjected to column chromatography (Non ion adsorption resin, Diaion HP 20 prepared by Mitsubishi Chemical Industries: 500 ml), eluting with water (0.5%), 5% (1 l) and 15% (2.5 l) isopropyl alcohol. The fractions containing the object compound were collected and then concentrated. The concentrate was lyophilized to give 7-[2-methoxyimino-2-(2-aminothiazol-4-yl)acetamido]-3-[1-[3-(4-methyl-1-piperazinyl)propyl]-1H-tetrazol-5-yl]thiomethyl-3-cephem-4-carboxylic acid (syn isomer). (9.2 g).

I.R. (Nujol): 3300, 1760, 1660, 1600, 1530 $cm^{-1}$.

N.M.R. (DMSO-$d_6$, δ): 2.0 (2H, m), 2.3—3.0 (10H, m), 2.55 (3H, s), 3.47, 3.73 (2H, ABq J=18Hz), 3.84 (3H, s), 4.32 (4H, broad s), 5.05 (1H, d, J=5Hz), 5.67 (1H, dd, J=5 and 8 Hz), 6.75 (1H, s), 7.22 (2H, broad s), 9.51 (1H, d, J=8Hz).

**Claims for the Contracting States: BE CH DE FR GB IT LI NL SE**

1. A 1-substituted-1H-tetrazole-5-thiol compound of the formula:

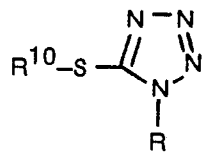

(III′)

wherein R is a group of the formula: $-A_1-R^{3a}$
[in which

$A_1$ is hydroxy($C_1-C_6$)alkylene, amino($C_1-C_6$)alkylene, protected amino ($C_1-C_6$)alkenylene, alkylene with up to 6C-atoms;

or hydroxyimino($C_1-C_6$)alkylene
wherein the hydrogen atom of the hydroxyimino group may be replaced with a ($C_1-C_6$) aliphatic hydrocarbon group; and

$R^{3a}$ is carboxy or an esterified carboxy group] or a group of the formula:

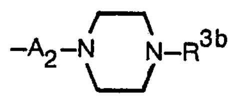

[in which

$A_2$ is ($C_1-C_6$)alkylene which may have an oxo group; and

$R^{3b}$ is carboxy, esterified carboxy, an aliphatic or armomatic group] and

$R^{10}$ is hydrogen or a mercapto-protective group, and a salt thereof.

2. A process for preparing a compound of formula (III) as defined in claim 1 which comprises

1) reacting a compound of the formula:

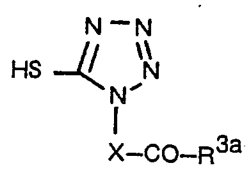

(IV)

wherein X is ($C_1-C_6$)alkylene and $R^{3a}$ is defined in claim 1; or its reactive derivative at the mercapto group or a salt thereof, with a compound of the formula:

$$H_2N-OR^4 \qquad (VIII)$$

wherein $R^4$ is hydrogen or a ($C_1-C_6$)aliphatic hydrocarbon group; or a salt thereof, to give a compound of the formula

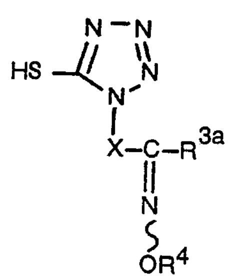

(IIIa)

wherein X, $R^{3a}$ and $R^4$ are each as defined above; or its reactive derivative at the mercapto group or a salt thereof; or

2) reducing a compound of the formula:

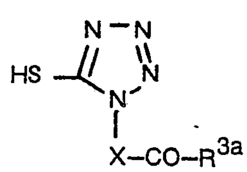

(IV)

wherein X and $R^{3a}$ are each as defined above; or its reactive derivative at the mercapto group or a salt thereof, to give a compound of the formula:

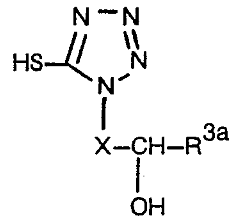

(IIIb)

wherein X and $R^{3a}$ are each as defined above; or its reactive derivative at the mercapto group or a salt thereof; or

3) reducing a compound of the formula:

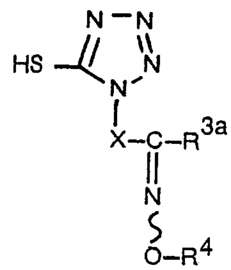

(IIIa)

wherein X, $R^{3a}$ and $R^4$ are each as defined above; or its reactive derivative at the mercapto group or a salt thereof, to give a compound of the formula:

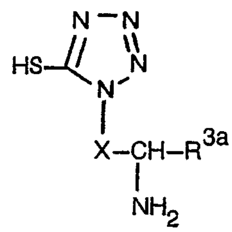

(IIIb)

wherein X and $R^{3a}$ are each as defined above; or its reactive derivative at the mercapto group or a salt thereof; or

4) subjecting a compound of the formula:

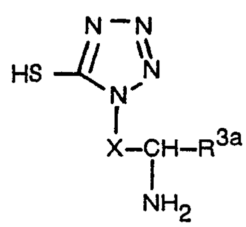

wherein X and $R^{3a}$ are as defined above; or its reactive derivative at the mercapto group or a salt thereof, to the introduction reaction of the amino protective group, to give a compound of the formula:

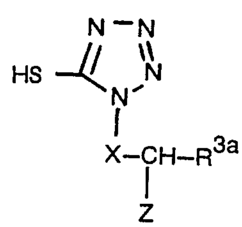

wherein X and $R^{3a}$ are each as defined above; and Z is a protected amino group; or its reactive derivative at the mercapto group or a salt thereof; or

5) subjecting a compound of the formula:

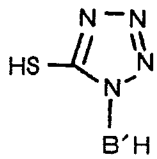

(If)

wherein B′ is ($C_2$—$C_6$)alkenylene, or its reactive derivative at the mercapto group, to the reaction to introduce a carboxy group, to give a compound of the formula:

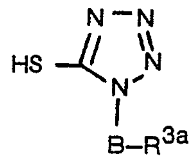

wherein $R^{3a}$ is as defined above; and B is ($C_2$—$C_6$)alkenylene or its reactive derivative at the mercapto group or a salt thereof; or

6) reacting a compound of the formula

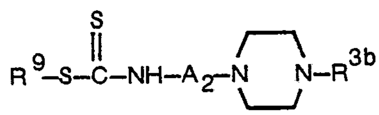

(XIII)

wherein $A_2$ and $R^{3b}$ are each as defined in claim 1, and $R^9$ is ($C_1$—$C_6$)alkyl or a salt thereof, with a hydrazoic acid salt (XII), to give a compound of the formula:

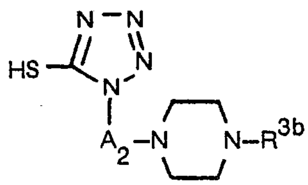

(IIIg)

wherein $A_2$ and $R^{3b}$ are each as defined above, or a salt thereof; or

7) reacting a compound of the formula:

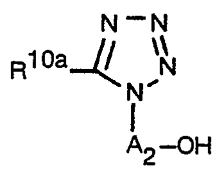

(XXI)

wherein $A_2$ is as defined above, and $R^{10a}$ is a mercapto protective group, or its reactive derivative at the hydroxy group, with a compound of the formula:

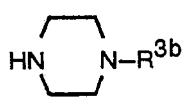

(XIX)

wherein $R^{3b}$ is as defined above, or a salt thereof, to give a compound of the formula:

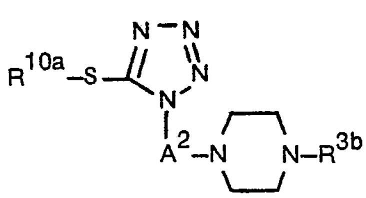

(XX)

wherein $A_2$, $R^{3b}$ and $R^{10a}$ are each as defined above, or a salt thereof; or

8) subjecting the compound of the formula:

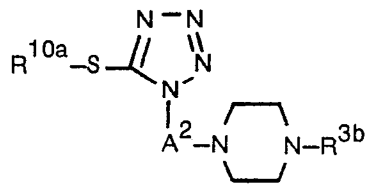

(XX)

wherein $A_2$, $R^{3b}$ and $R^{10a}$ are each as defined above, or a salt thereof, to the elimination reaction of the mercapto-protective group, to give a compound of the formula:

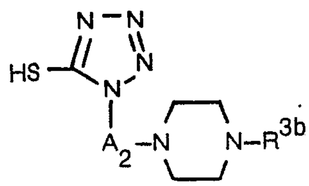

(IIIg)

wherein $A_2$ and $R^{3b}$ are each as defined above, or a salt thereof; or

9) reacting a compound of the formula:

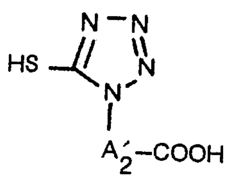

(XXV)

wherein $A_2'$ is $(C_1—C_6)$alkylene, or its reactive derivative at the carboxy group or a salt thereof, with the compound of the formula:

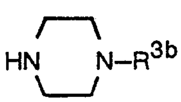

(XIX)

wherein $R^{3b}$ is as defined above, or its reactive derivative at the amino group or a salt thereof, to give a compound of the formula:

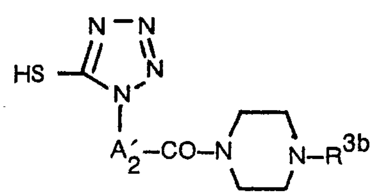

(IIIi)

wherein $A_2'$ and $R^{3b}$ are each as defined above, or a salt thereof.

**Claim for the Contracting State: AT**

A process for preparing a 1-substituted-1H-tetrazole-5-thiol compound of the formula:

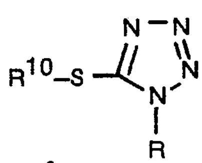

(III')

wherein R is a group of the formula: $—A_1—R^{3a}$

[in which $A_1$ is hydroxy$(C_1—C_6)$alkylene, amino$(C_1—C_6)$alkylene, protected amino$(C_1—C_6)$alkylene, alkenylene with up to 6C-atoms;

or hydroxyimino$(C_1—C_6)$alkylene

wherein the hydrogen atom of the hydroxyimino group may be replaced with a $(C_1—C_6)$ aliphatic hydrocarbon group; and

$R^{3a}$ is carboxy or an esterified carboxy group] or a group of the formula:

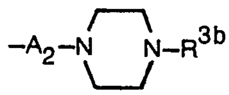

[in which $A_2$ is $(C_1—C_6)$alkylene which may have an oxo group; and

$R^{3b}$ is carboxy, esterified carboxy, an aliphatic or aromatic group] and

$R^{10}$ is hydrogen or a mercapto-protective group, or a salt thereof, which comprises

1) reacting a compound of the formula:

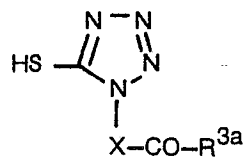
(IV)

wherein X and $R^{3a}$ are each as defined above; or its reactive derivative at the mercapto group or a salt thereof, with a compound of the formula:

$$H_2N—OR^4 \qquad (VIII)$$

wherein $R^4$ is hydrogen or a $(C_1—C_6)$aliphatic hydrocarbon group; or a salt thereof, to give a compound of the formula:

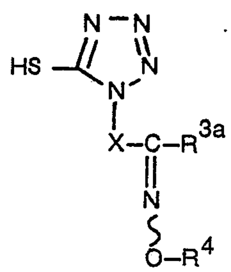
(IIIa)

wherein X, $R^{3a}$ and $R^4$ are each as defined above; or its reactive derivative at the mercapto group or a salt thereof; or

2) reducing a compound of the formula:

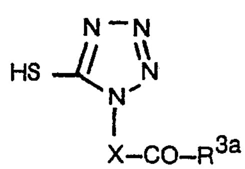
(IV)

wherein X and $R^{3a}$ are each as defined above; or its reactive derivative at the mercapto group or a salt thereof; to give a compound of the formula:

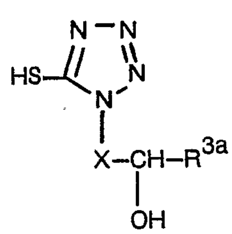
(IIIb)

wherein X and $R^{3a}$ are each as defined above; or its reactive derivative at the mercapto group or a salt thereof; or

3) reducing a compound of the formula:

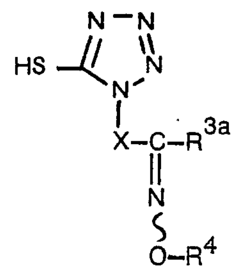

(IIIa)

wherein X, $R^{3a}$ and $R^4$ are each as defined above; or its reactive derivative at the mercapto group or a salt thereof, to give a compound of the formula:

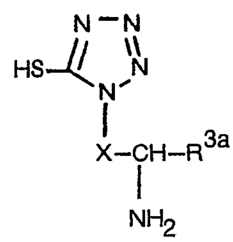

(IIIb)

wherein X and $R^{3a}$ are each as defined above; or its reactive derivative at the mercapto group or a salt thereof; or

4) subjecting a compound of the formula:

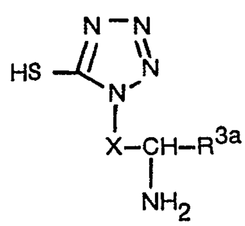

wherein X and $R^{3a}$ are as defined above; or its reactive derivative at the mercapto group or a salt thereof, to the introduction reaction of the amino protective group, to give a compound of the formula:

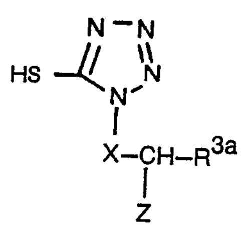

wherein X and $R^{3a}$ are each as defined above; and Z is a protected amino group; or its reactive derivative at the mercapto group or a salt thereof; or

5) subjecting a compound of the formula:

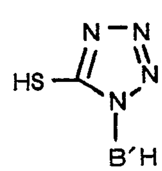

(If)

wherein B′ is $(C_2—C_6)$alkenylene, or its reactive derivative at the mercapto group, to the reaction to introduce a carboxy group, to give a compound of the formula:

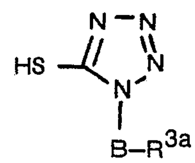

wherein $R^{3a}$ is as defined above; and B is $(C_2—C_6)$alkenylene or its reactive derivative at the mercapto group or a salt thereof; or

6) reacting a compound of the formula:

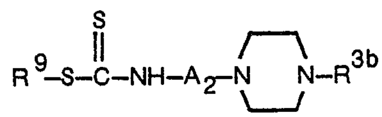 (XIII)

wherein $A_2$ and $R^{3b}$ are each as defined above, and $R^9$ is $(C_1—C_6)$alkyl or a salt thereof, with a hydrazoic acid salt (XII), to give a compound of the formula:

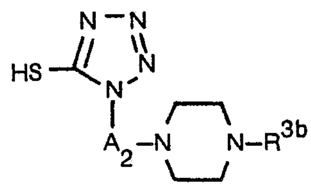 (IIIg)

wherein $A_2$ and $R^{3b}$ are each as defined above, or a salt thereof; or

7) reacting a compound of the formula:

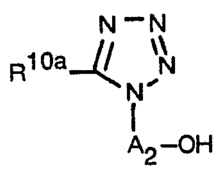 (XXI)

wherein $A_2$ is as defined above, and $R^{10a}$ is a mercapto protective group, or its reactive derivative at the hydroxy group, with a compound of the formula:

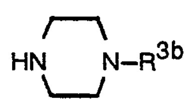 (XIX)

wherein $R^{3b}$ is as defined above, or a salt thereof, to give a compound of the formula:

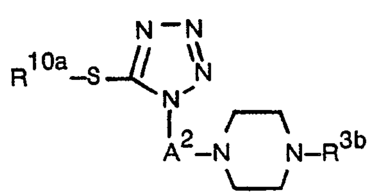 (XX)

wherein $A_2$, $R^{3b}$ and $R^{10a}$ are each as defined above, or a salt thereof; or

8) subjecting the compound of the formula:

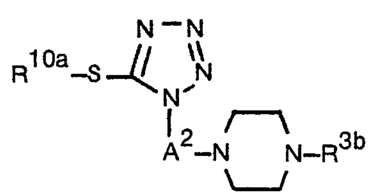 (XX)

wherein $A_2$, $R^{3b}$ and $R^{10a}$ are each as defined above, or a salt thereof, to the elimination reaction of the mercapto-protective group, to give a compound of the formula:

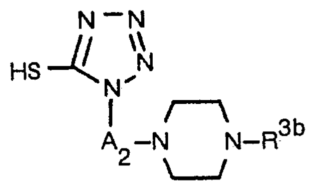

(IIIg)

wherein $A_2$ and $R^{3b}$ are each as defined above, or a salt thereof; or

9) reacting a compound of the formula:

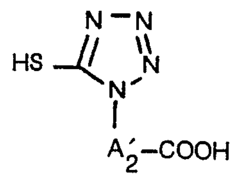

(XXV)

wherein $A_2'$ is $(C_1—C_6)$alkylene, or its reactive derivative at the carboxy group or a salt thereof, with the compound of the formula:

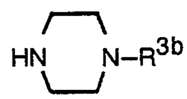

(XIX)

wherein $R^{3b}$ is as defined above; or its reactive derivative at the amino group or a salt thereof, to give a compound of the formula:

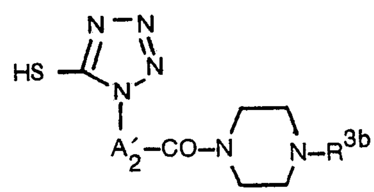

(IIIi)

wherein $A_2'$ and $R^{3b}$ are each as defined above, or a salt thereof.

**Patentansprüche für die Vertragsstaaten: BE CH DE FR GB IT LI NL SE**

1. 1-substituierte-1H-Tetrazol-5-thiol-Verbindung der Formel

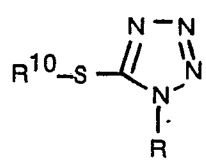

(III′)

worin R eine Gruppe der Formel $—A_1—R^{3a}$

[worin $A_1$ Hydroxy-$(C_1—C_6)$-alkylen, Amino-$(C_1—C_6)$-alkylen, geschütztes Amino-$(C_1—C_6)$-alkylen, Alkenylen mit bis zu 6 C-Atomen; oder Hydroxyimino-$(C_1—C_6)$-alkylen, worin das Wasserstoffatom der Hydroxyiminogruppe durch eine $(C_1—C_6)$-aliphatische Kohlenwasserstoffgruppe ersetzt sein kann, ist; und

$R^{3a}$ Carboxy oder eine veresterte Carboxygruppe ist] oder eine Gruppe der Formel

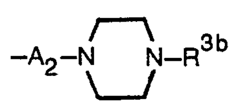

ist

[worin $A_2$ $(C_1—C_6)$-Alkylen ist, welches eine Oxogruppe aufweisen kann; und

$R^{3b}$ Carboxy, verestertes Carboxy, eine aliphatische oder aromatische Gruppe ist] und

$R^{10}$ Wasserstoff oder eine Mercapto-Schutzgruppe ist, und ein Salz davon.

2. Verfahren zur Herstellung einer Verbindung der Formel (III′), wie in Anspruch 1 definiert, durch

1) Reagieren einer Verbindung der Formel

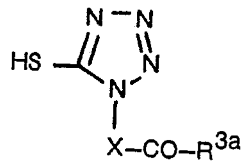

(IV)

worin X ($C_1$—$C_6$)-Alkylen ist und $R^{3a}$ wie in Anspruch 1 definiert ist; oder ihres reaktiven Derivats an der Mercaptogruppe oder eines Salzes davon mit einer Verbindung der Formel

$H_2N—OR^4$ (VIII)

worin $R^4$ Wasserstoff oder eine ($C_1$—$C_6$)-aliphatische Kohlenwasserstoffgruppe ist; oder einem Salz davon unter Bildung einer Verbindung der Formel

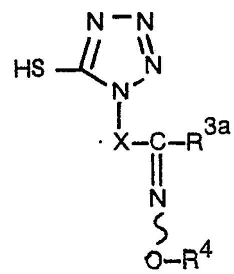

(IIIa)

worin X, $R^{3a}$ und $R^4$ jeweils wie oben definiert sind; oder ihres reaktiven Derivats an der Mercaptogruppe oder eines Salzes davon; oder

2) Reduzieren einer Verbindung der Formel

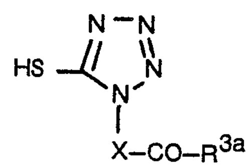

(IV)

worin X und $R^{3a}$ jeweils wie oben definiert sind; oder ihres reaktiven Derivats an der Mercaptogruppe oder eines Salzes davon unter Bildung einer Verbindung der Formel

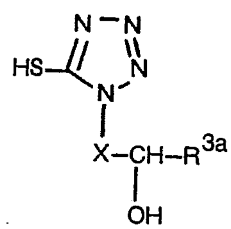

(IIIb)

worin X und $R^{3a}$ jeweils wie oben definiert sind; oder ihres reaktiven Derivats an der Mercaptogruppe oder eines Salzes davon; oder

3) Reduzieren einer Verbindung der Formel

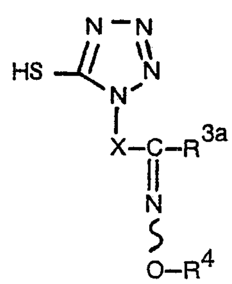

(IIIa)

worin X, $R^{3a}$ und $R^4$ jeweils wie oben definiert sind; oder ihres reaktiven Derivats an der Mercaptogruppe oder eines Salzes davon unter Bildung einer Verbindung der Formel

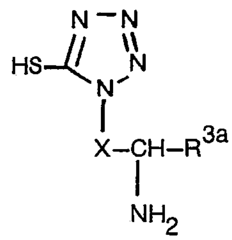

(IIIb)

worin X und $R^{3a}$ jeweils wie oben definiert sind; oder ihres reaktiven Derivats an der Mercaptogruppe oder eines Salzes davon; oder

4) Unterwerfen einer Verbindung der Formel

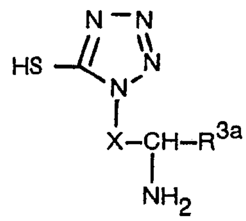

worin X und $R^{3a}$ jeweils wie oben definiert sind; oder ihres reaktiven Derivats an der Mercaptogruppe oder eines Salzes davon der Einführungsreaktion der Aminoschutzgruppe unter Bildung einer Verbindung der Formel

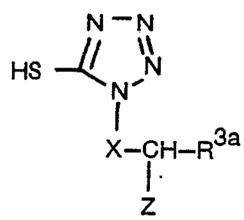

worin X und $R^{3a}$ jeweils wie oben definiert sind; und Z eine geschützte Aminogruppe ist; oder ihres reaktiven Derivats an der Mercaptogruppe oder eines Salzes davon; oder

5) Unterwerfen einer Verbindung der Formel

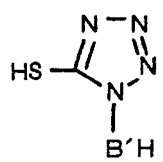

(If)

worin B′ $(C_2—C_6)$-Alkenylen ist, oder ihres reaktiven Derivats an der Mercaptogruppe der Reaktion zur Einführung einer Carboxygruppe unter Bildung einer Verbindung der Formel

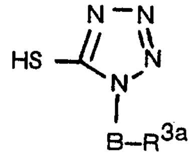

worin $R^{3a}$ wie oben definiert ist; und B $(C_2—C_6)$-Alkenylen ist oder ihres reaktiven Derivats an der Mercaptogruppe oder eines Salzes davon; oder

6) Reagieren einer Verbindung der Formel

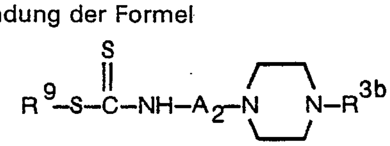

(XIII)

worin $A_2$ und $R^{3b}$ jeweils wie in Anspruch 1 definiert sind und $R^9$ ($C_1$—$C_6$)-Alkyl ist oder eines Salzes davon mit einem Salz der Stickstoffwasserstoffsäure (XII) unter Bildung einer Verbindung der Formel

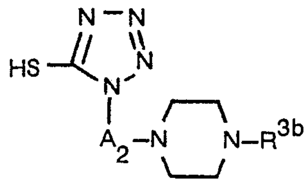

(IIIg)

worin $A_2$ und $R^{3b}$ jeweils wie oben definiert sind, oder eines Salzes davon; oder

7) Reagieren einer Verbindung der Formel

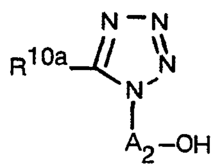

(XXI)

worin $A_2$ wie oben definiert ist und $R^{10a}$ eine Mercaptoschutzgruppe ist, oder ihres reaktiven Derivats an der Hydroxygruppe mit einer Verbindung der Formel

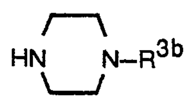

(XIX)

worin $R^{3b}$ wie oben definiert ist, oder einem Salz davon unter Bildung einer Verbindung der Formel

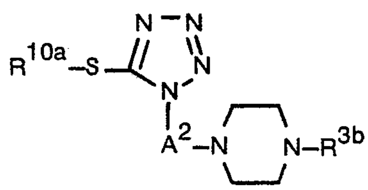

(XX)

worin $A_2$, $R^{3b}$ und $R^{10a}$ jeweils wie oben definiert sind, oder eines Salzes davon; oder

8) Unterwerfen der Verbindung der Formel

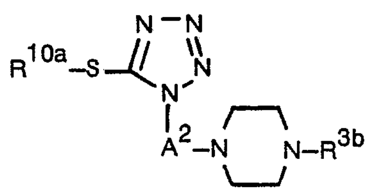

(XX)

worin $A_2$, $R^{3b}$ und $R^{10a}$ jeweils wie oben definiert sind, oder eines Salzes davon, der Eliminierungsreaktion für die Mercaptoschutzgruppe unter Bildung einer Verbindung der Formel

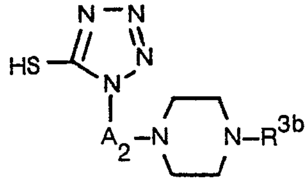

(IIIg)

worin $A_2$ und $R^{3b}$ jeweils wie oben definiert sind, oder eines Salzes davon; oder

9) Reagieren einer Verbindung der Formel

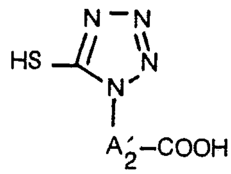

(XXV)

worin $A_2'$ ($C_1$—$C_6$)-Alkylen ist, oder ihres reaktiven Derivats an der Carboxygruppe oder eines Salzes davon mit der Verbindung der Formel

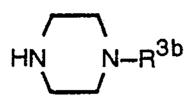

(XIX)

worin $R^{3b}$ wie oben definiert ist, oder ihrem reaktiven Derivat an der Aminogruppe oder einem Salz davon unter Bildung einer Verbindung der Formel

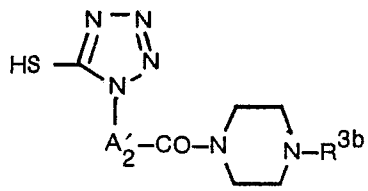

(IIIi)

worin $A_2'$ und $R^{3b}$ jeweils wie oben definiert sind, oder eines Salzes davon.

**Patentanspruch für den Vertragsstaat: AT**

Verfahren zur Herstellung einer 1-substituierten-1H-Tetrazol-5-thiolverbindung der Formel:

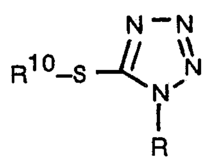

(III')

worin R eine Gruppe der Formel $—A_1—R^{3a}$
[worin $A_1$ Hydroxy-($C_1$—$C_6$)-alkylen, Amino-($C_1$—$C_6$)-alkylen, geschütztes Amino-($C_1$—$C_6$)-alkylen, Alkenylen mit bis zu 6 C-Atomen; oder Hydroxyimino-($C_1$—$C_6$)-alkylen, worin das Wasserstoffatom der Hydroxyiminogruppe durch eine ($C_1$—$C_6$)-aliphatische Kohlenwasserstoffgruppe ersetzt sein kann, ist; und
$R^{3a}$ Carboxy oder eine veresterte Carboxygruppe ist] oder
eine Gruppe der Formel

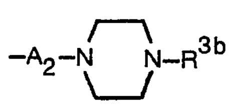

ist
[worin $A_2$ ($C_1$—$C_6$)-Alkylen ist, welches eine Oxogruppe aufweisen kann; und
$R^{3b}$ Carboxy, verestertes Carboxy, eine aliphatische oder aromatische Gruppe ist] und
$R^{10}$ Wasserstoff oder eine Mercapto-Schutzgruppe ist, oder eines Salzes davon durch
1) Reagieren einer Verbindung der Formel

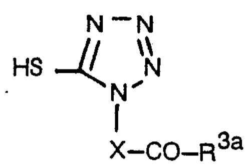

(IV)

worin X ($C_1$—$C_6$)-Alkylen ist und $R^{3a}$ wie in Anspruch 1 definiert ist; oder ihres reaktiven Derivats an der

Mercaptogruppe oder eines Salzes davon mit einer Verbindung der Formel

$$H_2N—OR^4 \qquad (VIII)$$

worin $R^4$ Wasserstoff oder eine $(C_1—C_6)$-aliphatische Kohlenwasserstoffgruppe ist; oder einem Salz davon under Bildung einer Verbindung der Formel

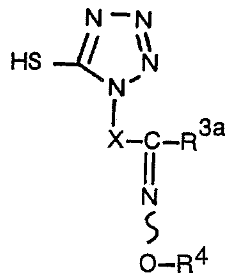

(IIIa)

worin X, $R^{3a}$ und $R^4$ jeweils wie oben definiert sind; oder ihres reaktiven Derivats an der Mercaptogruppe oder eines Salzes davon; oder

2) Reduzieren einer Verbindung der Formel

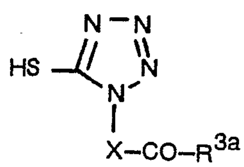

(IV)

worin X und $R^{3a}$ jeweils wie oben definiert sind; oder ihres reaktiven Derivats an der Mercaptogruppe oder eines Salzes davon unter Bildung einer Verbindung der Formel

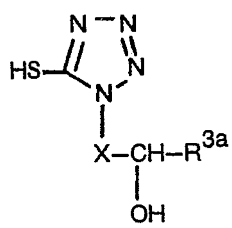

(IIIb)

worin X und $R^{3a}$ jeweils wie oben definiert sind; oder ihres reaktiven Derivats an der Mercaptogruppe oder eines Salzes davon; oder

3) Reduzieren einer Verbindung der Formel

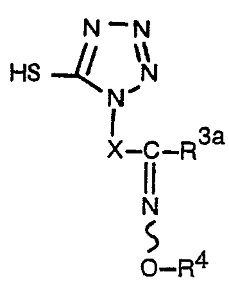

(IIIa)

worin X, $R^{3a}$ und $R^4$ jeweils wie oben definiert sind; oder ihres reaktiven Derivats an der Mercaptogruppe oder eines Salzes davon unter Bildung einer Verbindung der Formel

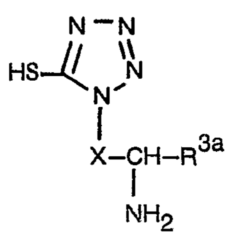

(IIIb)

worin X und $R^{3a}$ jeweils wie oben definiert sind; oder ihres reaktiven Derivats an der Mercaptogruppe oder eines Salzes davon; oder

4) Unterwerfen einer Verbindung der Formel

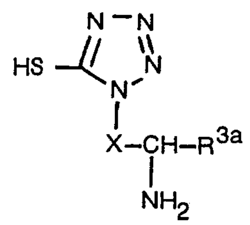

worin X und $R^{3a}$ jeweils wie oben definiert sind; oder ihres reaktiven Derivats an der Mercaptogruppe oder eines Salzes davon der Einführungsreaktion der Aminoschutzgruppe unter Bildung einer Verbindung der Formel

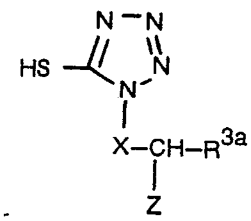

worin X und $R^{3a}$ jeweils wie oben definiert sind; und Z eine geschützte Aminogruppe ist; oder ihres reaktiven Derivats an der Mercaptogruppe oder eines Salze davon; oder

5) Unterwerfen einer Verbindung der Formel

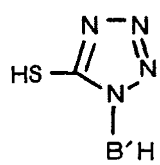

(If)

worin B′ ($C_2$—$C_6$)-Alkenylen ist, oder ihres reaktiven Derivats an der Mercaptogruppe der Reaktion zur Einführung einer Carboxygruppe unter Bildung einer Verbindung der Formel

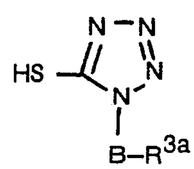

worin $R^{3a}$ wie oben definiert ist; und B ($C_2$—$C_6$)-Alkenylen ist oder ihres reaktiven Derivats an der Mercaptogruppe oder eines Salzes davon; oder

6) Reagieren einer Verbindung der Formel

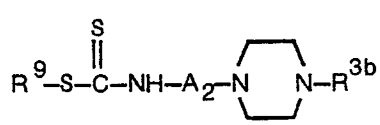

(XIII)

worin $A_2$ und $R^{3b}$ jeweils wie in Anspruch 1 definiert sind und $R^9$ ($C_1$—$C_6$)-Alkyl ist oder eines Salzes davon mit einem Salzes der Stickstoffwasserstoffsäure (XII) unter Bildung einer Verbindung der Formel

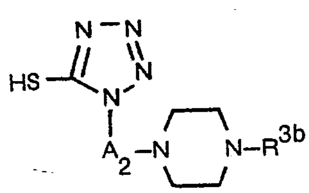

(IIIg)

worin $A_2$ und $R^{3b}$ jeweils wie oben definiert sind, oder eines Salzes davon; oder

7) Reagieren einer Verbindung der Formel

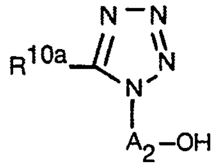

(XXI)

worin $A_2$ wie oben definiert ist und $R^{10a}$ eine Mercaptoschutzgruppe ist, oder ihres reaktiven Derivats an der Hydroxygruppe mit einer Verbindung der Formel

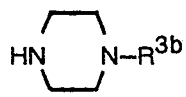

(XIX)

worin $R^{3b}$ wie oben definiert ist, oder einem Salz davon unter Bildung einer Verbindung der Formel

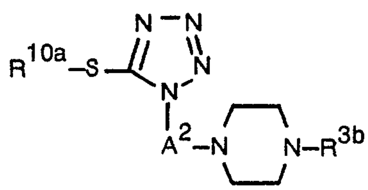

(XX)

worin $A_2$, $R^{3b}$ und $R^{10a}$ jeweils wie oben definiert sind, oder eines Salzes davon; oder

8) Unterwerfen der Verbindung der Formel

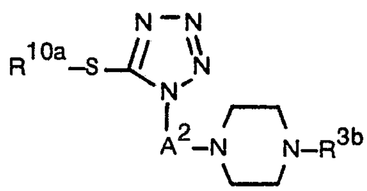

(XX)

worin $A_2$, $R^{3b}$ und $R^{10a}$ jeweils wie oben definiert sind, oder eines Salzes davon, der Eliminierungsreaktion für die Mercaptoschutzgruppe unter Bildung einer Verbindung der Formel

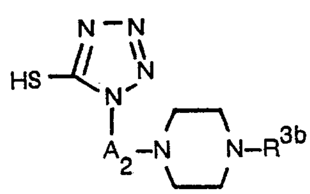

(IIIg)

worin $A_2$ und $R^{3b}$ jeweils wie oben definiert sind, oder eines Salzes davon; oder

9) Reagieren einer Verbindung der Formel

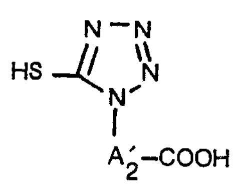

(XXV)

worin $A_2'$ ($C_1$—$C_6$)-Alkylen ist, oder ihres reaktiven Derivats an der Carboxygruppe oder eines Salzes davon mit der Verbindung der Formel

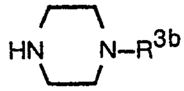

(XIX)

worin $R^{3b}$ wie oben definiert ist, oder ihrem reaktiven Derivat an der Aminogruppe oder einem Salz davon unter Bildung einer Verbindung der Formel

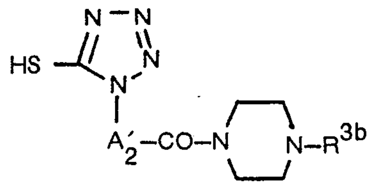

(IIIi)

worin $A_2'$ und $R^{3b}$ jeweils wie oben definiert sind, oder eines Salzes davon.

**Revendications pour les Etats contractants: BE CH DE FR GB IT LI LU NL SE**

1. Composé de 1-substitué-1H-tétrazole-5-thiol répondant à la formule:

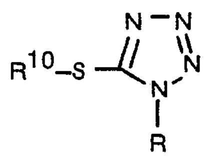

(III')

dans laquelle R est un groupe répondant à la formule: $—A_1—R^{3a}$ .
dans laquelle $A_1$ est un groupe hydroxy alkylène en $C_1—C_6$, un groupe amino alkylène en $C_1—C_6$, un groupe amino protégé alkylène en $C_1—C_6$; un groupe alcénylène ayant jusqu'à 6 atomes de carbone ou un groupe hydroxyimino alkylène en $C_1—C_6$, où l'atome d'hydrogène du groupe hydroxyimino peut être remplacé par un groupe hydrocarboné aliphatique en $C_1—C_6$; et

$R^{3a}$ est un groupe carboxy ou un groupe carboxy estérifié ou un groupe répondant à la formule:

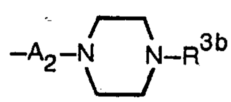

dans laquelle $A_2$ est un groupe alkylène en $C_1—C_6$ qui peut avoir un groupe oxo, et

$R^{3b}$ est un groupe carboxy, carboxy estérifié, aliphatique ou aromatique et

$R^{10}$ est l'hydrogène ou un groupe protecteur du groupe mercapto, et un de ses sels.

2. Procédé de préparation d'un composé répondant à la formule (III') tel que défini dans la revendication 1, qui comprend les étapes consistant à:

1) faire réagir un composé répondant à la formule:

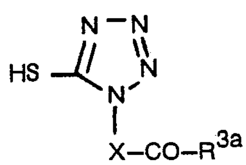

(IV)

dans laquelle X est un groupe alkylène en $C_1—C_6$ et $R^{3a}$ est tel que défini dans la revendication l'ou son dérivé réactif au groupe mercapto ou un de ses sels, avec un composé répondant à la formule:

$$H_2N—OR^4 \qquad \text{(VIII)}$$

dans laquelle $R^4$ est l'hydrogène ou un groupe hydrocarboné aliphatique en $C_1—C_6$ ou un de ses sels, afin d'obtenir un composé répondant à la formule:

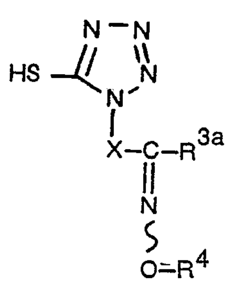

(IIIa)

dans laquelle X, $R^{3a}$ et $R^4$ sont chacun tels que définis ci-dessus, ou son dérivé réactif au groupe mercapto ou un de ses sels; ou

2) faire réagir un composé répondant à la formule:

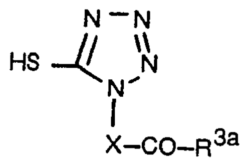

(IV)

dans laquelle X et $R^{3a}$ sont chacun tels que définis ci-dessus; ou son dérivé réactif au groupe mercapto ou un de ses sels, afin d'obtenir un composé répondant à la formule:

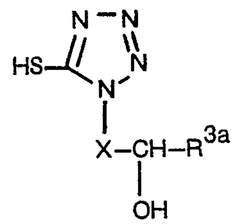

(IIIb)

dans laquelle X et $R^{3a}$ sont chacun tels que définis ci-dessus; ou son dérivé réactif au groupe mercapto ou un de ses sels; ou

3) réduire un composé répondant à la formule:

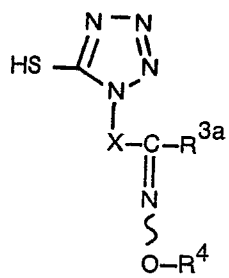

(IIIa)

dans laquelle X, $R^{3a}$ et $R^4$ sont chacun tels que définis ci-dessus, ou son dérivé réactif au groupe mercapto ou un de leurs sels, afin d'obtenir un composé répondant à la formule:

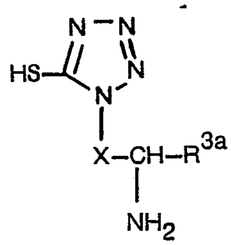

(IIIb)

dans laquelle X et $R^{3a}$ sont chacun tels que définis ci-dessus, ou son dérivé réactif au groupe mercapto ou un de ses sels; ou

4) à soumettre un composé répondant à la formule:

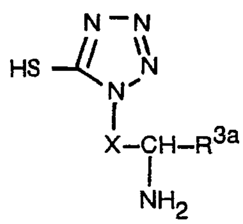

dans laquelle X et $R^3$ sont tels que définis ci-dessus, ou son dérivé réactif au groupe mercapto ou un de ses

sels, à la réaction d'introduction du groupe protecteur du groupe amino, afin d'obtenir un composé répondant à la formule:

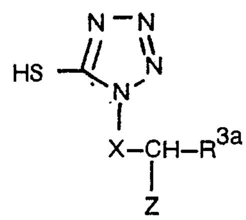

dans laquelle X et $R^{3a}$ sont chacun tels que définis ci-dessus, et Z est un groupe amino protégé; ou son dérivé réactif au groupe mercapto ou un de ses sels; ou

5) soumettre un composé répondant à la formule:

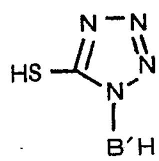

(If)

dans laquelle B' est un groupe alcénylène en $C_2$—$C_6$, ou son dérivé réactif au groupe mercapto, à la réaction afin d'introduire un groupe carboxy, dans le but d'obtenir un composé répondant à la formule:

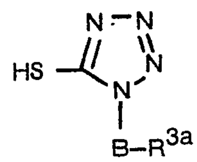

dans laquelle $R^{3a}$ est tel que défini ci-dessus, et B est un groupe alcénylène en $C_2$—$C_6$ ou son dérivé réactif au groupe mercapto ou un de ses sels; ou

6) faire réagir un composé répondant à la formule:

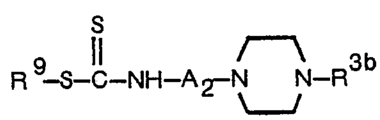

(XIII)

dans laquelle $A_2$ et $R^{3b}$ sont chacun tels que définis dans la revendication 1, et $R^9$ est un groupe alkyle en $C_1$—$C_6$ ou un de ses sels, avec un sel de l'acide hydrazoïque (XII), afin d'obtenir un composé répondant à la formule:

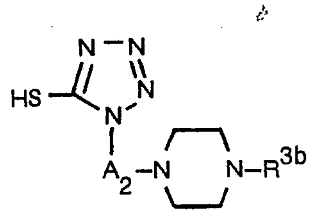

(IIIg)

dans laquelle $A_2$ et $R^{3b}$ sont chacun tels que définis ci-dessus ou un de ses sels; ou

7) faire réagir un composé répondant à la formule:

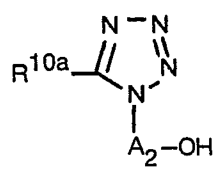

(XXI)

dans laquelle $A_2$ est tel que défini ci-dessus et $R^{10a}$ est un groupe protecteur du groupe mercapto, ou son dérivé réactif au groupe hydroxy, avec un composé répondant à la formule:

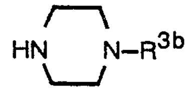

(XIX)

dans laquelle $R^{3b}$ est tel que défini ci-dessus, ou un de ses sels, afin d'obtenir un composé répondant à la formule:

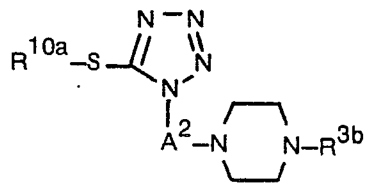

(XX)

dans laquelle $A_2$, $R^{3b}$ et $R^{10a}$ sont chacun tels que définis ci-dessus, ou un de ses sels, ou

8) soumettre le composé répondant à la formule:

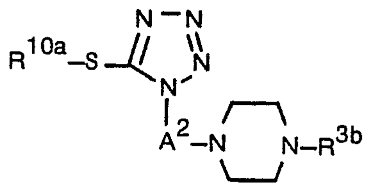

(XX)

dans laquelle $A_2$, $R^{3b}$ et $R^{10a}$ sont chacun tels que définis ci-dessus, ou un de ses sels, à la réaction d'élimination du groupe protecteur du groupe mercapto, afin d'obtenir un composé répondant à la formule:

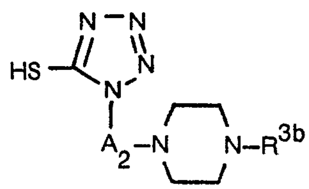

(IIIg)

dans laquelle $A_2$ et $R^{3b}$ sont chacun tels que définis ci-dessus, ou un de ses sels; ou

9) faire réagir un composé à la formule:

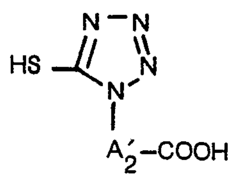

(XXV)

dans laquelle $A'_2$ est un groupe alkylène en $C_1$—$C_6$, ou son dérivé réactif au groupe carboxy ou un de ses sels, avec le composé répondant à la formule:

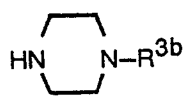

(XIX)

dans laquelle $R^{3b}$ est tel que défini ci-dessus, ou son dérivé réactif au groupe amino ou un de ses sels, afin d'obtenir un composé répondant à la formule:

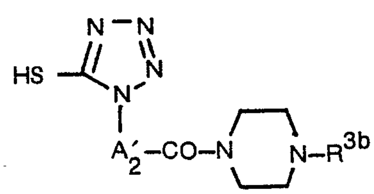

(IIIi)

dans laquelle $A'_2$ et $R^{3b}$ sont chacun tels que définis ci-dessus ou un de ses sels.

**Revendication pour l'Etat contractant: AT**

Procédé de préparation d'un composé de 1-substitué-1H-tétrazole-5-thiol, répondant à la formule:

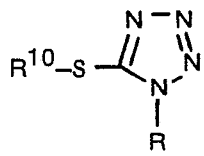

(III′)

dans laquelle R est un groupe répondant à la formule: $A_1$—$R^{3a}$
dans laquelle $A_1$ est un groupe hydroxyalkylène en $C_1$—$C_6$, un groupe amino alkylène en $C_1$—$C_6$, un groupe amino protégé alkylène en $C_1$—$C_6$, un groupe alcénylène ayant jusqu'à 6 atomes de carbone,
ou un groupe hydroxyimino, alkylène en $C_1$—$C_6$, dans lequel l'atome d'hydrogène du groupe hydroxyimino peut être remplacé par un groupe hydrocarboné aliphatique en $C_1$—$C_6$; et
$R^{3a}$ est un groupe carboxy ou un groupe carboxy estérifié ou un groupe répondant à la formule:

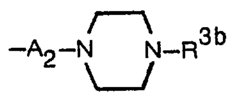

dans laquelle $A_2$ est un groupe alkylène en $C_1$—$C_6$ qui peut avoir un groupe oxo; et
$R^{3b}$ est un groupe carboxy, carboxy estérifié, un groupe aliphatique ou aromatique et
$R^{10}$ est l'hydrogène ou un groupe protecteur du groupe mercapto ou un de ses sels, qui consiste à
1) faire réagir un composé répondant à la formule:

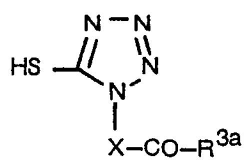

(IV)

dans laquelle X et $R^{3a}$ sont chacun tels que définis ci-dessus ou son dérivé réactif au groupe mercapto ou un de ses sels, avec un composé répondant à la formule:

$$H_2N—OR^4 \quad (VIII)$$

dans laquelle $R^4$ est l'hydrogène ou un groupe hydrocarboné aliphatique en $C_1$—$C_6$ ou un de ses sels afin d'obtenir un composé répondant à la formule:

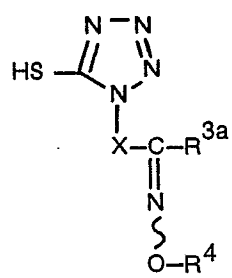

(IIIa)

dans laquelle X, $R^{3a}$ et $R^4$ sont chacun tels que définis ci-dessus, ou son dérivé réactif au groupe mercapto ou un de ses sels, ou
2) réduire un composé répondant à la fórmule:

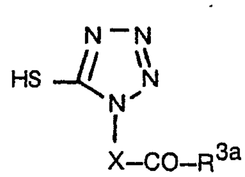

(IV)

dans laquelle X et $R^{3a}$ sont chacun tels que définis ci-dessus, ou son dérivé réactif au groupe mercapto ou un de ses sels, afin d'obtenir un composé répondant à la formule:

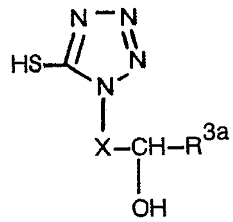

(IIIb)

dans laquelle X et $R^{3a}$ sont chacun tels que définis ci-dessus ou son dérivé réactif au groupe mercapto ou un de ses sels; ou

3) réduire un composé répondant à la formule:

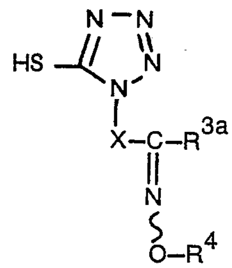

(IIIa)

dans laquelle X, $R^{3a}$ et $R^4$ sont chacun tels que définis ci-dessus; ou son dérivé réactif au groupe mercapto ou un de ses sels, afin d'obtenir un composé répondant à la formule:

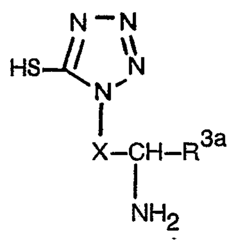

(IIIb)

dans laquelle X et $R^{3a}$ sont chacun tels que définis ci-dessus ou son dérivé réactif au groupe mercapto ou un de ses sels; ou

4) soumettre un composé répondant à la formule:

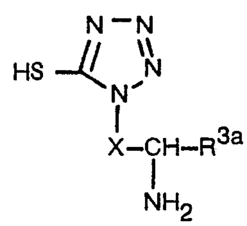

dans laquelle X et $R^{3a}$ sont chacun tels que définis ci-dessus, ou son dérivé réactif au groupe mercapto ou un de ses sels, à la réaction d'introduction du groupe protecteur du groupe amino, afin d'obtenir un composé répondant à la formule:

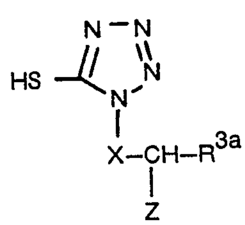

dans laquelle X et $R^{3a}$ sont chacun tels que définis ci-dessus; et Z est un groupe amino protégé; ou son dérivé réactif au groupe mercapto ou un de ses sels; ou

5) soumettre un composé répondant à la formule:

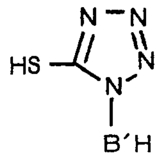

(If)

dans laquelle B′ est un groupe alcénylène en $C_2$—$C_6$, ou son dérivé réactif au groupe mercapto, à la réaction afin d'introduire un groupe carboxy, pour obtenir un composé répondant à la formule:

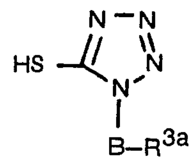

dans laquelle $R^{3a}$ est tel que défini ci-dessus, et B est un groupe alcénylène en $C_2$—$C_6$ ou son dérivé réactif au groupe mercapto ou un de ses sels, ou

6) faire réagir un composé répondant à la formule:

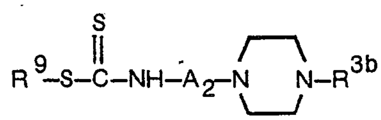

(XIII)

dans laquelle $A_2$ et $R^{3b}$ sont chacun tels que définis ci-dessus, et $R^9$ est un groupe alkyle en $C_1$—$C_6$ ou un de ses sels, avec un sel de l'acide hydrazoïque (XII), afin d'obtenir un composé répondant à la formule:

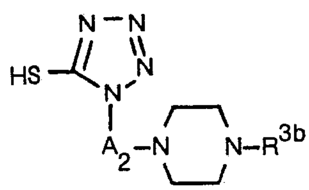

(IIIg)

dans laquelle $A_2$ et $R^{3b}$ sont chacun tels que définis ci-dessus, ou un de ses sels, ou

7) faire réagir un composé répondant à la formule:

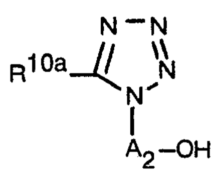

(XXI)

dans laquelle $A_2$ est tel que défini ci-dessus, et $R^{10a}$ est un groupe protecteur du groupe mercapto, ou son dérivé réactif au groupe hydroxy, avec un composé répondant à la formule:

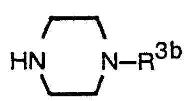

(XIX)

dans laquelle $R^{3b}$ est tel que défini ci-dessus, ou un de ses sels, afin d'obtenir un composé répondant à la formule:

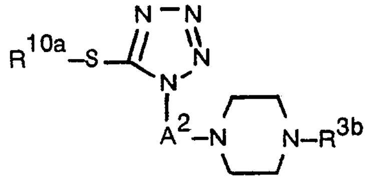

(XX)

dans laquelle $A_2$, $R^{3b}$ et $R^{10a}$ sont chacun tels que définis ci-dessus, ou un de ses sels; ou

8) soumettre le composé répondant à la formule:

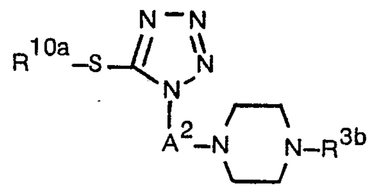

(XX)

dans laquelle $A_2$, $R^{3b}$ et $R^{10a}$ sont chacun tels que définis ci-dessus, ou un de ses sels, à la réaction d'élimination du groupe protecteur du groupe mercapto, afin, d'obtenir un composé répondant à la formule:

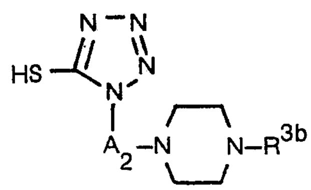

(IIIg)

dans laquelle $A_2$ et $R^{3b}$ sont chacun tels que définis ci-dessus ou un de ses sels, ou

9) faire réagir un composé répondant à la formule:

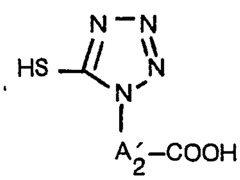

(XXV)

dans laquelle $A'_2$ est un groupe alkylène en $C_1$—$C_6$, ou son dérivé réactif au groupe carboxy ou un de ses sels, avec le composé répondant à la formule:

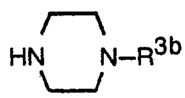

(XIX)

dans laquelle $R^{3b}$ est tel que défini ci-dessus, ou son dérivé réactif au groupe amino ou un de ses sels, afin d'obtenir un composé répondant à la formule:

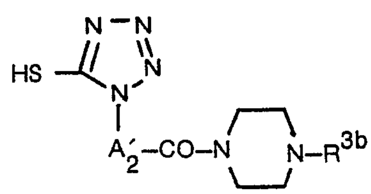

(IIIi)

dans laquelle $A'_2$ et $R^{3b}$ sont chacun tels que définis ci-dessus, ou un de ses sels.